# FASCICULE DE BREVET EUROPEEN

(11) **EP 1 115 719 B1**
(45) Date de publication et mention de la délivrance du brevet: **05.03.2003**
(21) Numéro de dépôt: 99943024.2
(22) Date de dépôt: 22.09.1999
(51) Int. Cl.: C07D 333/36, A61K 31/38, A61K 31/495, C07D 409/12, C07D 333/38

(54) **DERIVES D'AMIDINES, LEUR PREPARATION, LEUR APPLICATION A TITRE DE MEDICAMENTS ET LES COMPOSITIONS PHARMACEUTIQUES LES CONTENANT**
AMIDINDERIVATE, IHRE HERSTELLUNG, IHRE VERWENDUNG IN MEDIKAMENTEN UND SIE ENTHALTENDE PHARMAZEUTISCHE ZUBEREITUNGEN
NOVEL AMIDINE DERIVATIVES, THEIR PREPARATION AND APPLICATION AS MEDICINES AND PHARMACEUTICAL COMPOSITIONS CONTAINING SAME

(30) Priorité: 23.09.1998 FR 9811868
(43) Date de publication de la demande: 18.07.2001
(60) Demande divisionnaire: 02026170.7
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUVIN, Serge, F-91730 Mauchamps (FR); CHABRIER DE LASSAUNIERE, Pierre-Etienne, F-75016 Paris (FR); HARNETT, Jeremiah, F-91190 Gif-sur-Yvette (FR); PONS, Dominique, F-75014 Paris (FR); ULIBARRI, Gérard, F-91440 Bures-sur-Yvette (FR)
(74) Mandataire: Bourgouin, André
(86) Numéro de dépôt international: FR9902250
(87) Numéro de publication internationale: WO00017190

(56) Documents cités:
- EP-A- 0 782 986
- EP-A- 0 798 292
- WO-A-95/00505
- WO-A-95/05363
- WO-A-96/01817
- WO-A-97/12860
- WO-A-98/42696
- CHEMICAL ABSTRACTS, vol. 127, no. 6, 11 août 1997 (1997-08-11) Columbus, Ohio, US; abstract no. 86214, ASLAM, SOHAIL ET AL: "Preparation and analysis of dulcin-p-amino benzoic acid complex" XP002107677 & PAK. J. PHARM. (LAHORE) (1995), 8(2), 9-22 CODEN: PAJPEN;ISSN: 1019-956X,
- CHEMICAL ABSTRACTS, vol. 104, no. 7, 17 février 1986 (1986-02-17) Columbus, Ohio, US; abstract no. 47665, BAJUSZ, S.: "Interaction of trypsin-like enzymes with small inhibitors" XP002107678 & SYMP. BIOL. HUNG. (1984), 25(PROTEINASE ACTION), 277-98 CODEN: SYBHAK;ISSN: 0082-0695,

## Description

La présente invention a pour objet des nouveaux dérivés d'amidines, lesquels présentent une activité inhibitrice des enzymes NO-synthases produisant le monoxyde d'azote NO et / ou une activité piégeuse des formes réactives de l'oxygène (ROS pour "*reactive oxygen species*"). L'invention concerne en particulier les dérivés correspondant à la formule générale (I') définie ci-après, leurs méthodes de préparation, les préparations pharmaceutiques les contenant et leur utilisation à des fins thérapeutiques, en particulier leur utilisation en tant qu'inhibiteurs des NO-synthases et piégeurs de formes réactives de l'oxygène de manière sélective ou non.

Compte tenu du rôle potentiel du NO et des ROS en physiopathologie, les nouveaux dérivés décrits répondant à la formule générale (I') peuvent produire des effets bénéfiques ou favorables dans le traitement de pathologies où ces espèces chimiques sont impliquées. Notamment :
- Les maladies inflammatoires et prolifératives comme par exemple l'athérosclérose, l'hypertension pulmonaire, la détresse respiratoire, la glomérulonéphrite, l'hypertension portale, le psoriasis, l'arthrose et l'arthrite rhumatoïde, les fibroses, l'angiogenèse, les amyloïdoses, les inflammations du système gastro-intestinal (colite ulcérative ou non, maladie de Crohn), les diarrhées.
- Les maladies affectant le système pulmonaire ou les voies aériennes (asthme, sinusites, rhinites).
- Les maladies cardio-vasculaires et cérébro-vasculaires comprenant par exemple la migraine, l'hypertension artérielle, le choc septique, les infarctus cardiaques ou cérébraux d'origine ischémique ou hémorragiques, les ischémies et les thromboses.
- Les troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d'Alzheimer, la chorée de Huntington, la maladie de Parkinson, la maladie de Creutzfeld Jacob et les maladies à prions, la sclérose latérale amyotrophique ; les neuropathies oculaires comme le glaucome mais aussi la douleur, les traumatismes cérébraux ou de la moelle épinière, l'addiction aux opiacées, à l'alcool et aux substances induisant une accoutumance, les désordres cognitifs, les encéphalopathies, les encéphalopathies d'origine virale ou toxique.
- Les troubles du muscle squelettique et des jonctions neuromusculaires (myopathie, myosite) ainsi que les maladies cutanées.
- La cataracte.
- Les transplantations d'organes.
- Les maladies auto-immunes et virales comme par exemple le lupus, le sida, les infections parasitaires et virales, le diabète et ses complications, la sclérose en plaques.
- Le cancer.
- Les maladies neurologiques associées à des intoxications (empoisonnement au Cadmium, inhalation de n-hexane, pesticide, herbicide), à des traitements (radiothérapie) ou à des désordres d'origine génétique (maladie de Wilson).
- Toutes les pathologies caractérisées par une production excessive ou un dysfonctionnement de NO et/ou des ROS.

Dans l'ensemble de ces pathologies, il existe des évidences expérimentales démontrant l'implication du NO ou des ROS (*J. Med. Chem.* (1995) **38**, 4343-4362 ; *Free Radic. Biol. Med.* (1996) **20**, 675-705 ; *The Neuroscientist* (1997) **3**, 327-333).

Par ailleurs les inventeurs ont déjà décrit dans des brevets antérieurs des inhibiteurs de NO Synthases et leur utilisation (brevets US 5,081,148 ; US 5,360,925), et plus récemment l'association de ces inhibiteurs avec des produits possédant des propriétés antioxydantes ou antiradicalaires (demande de brevet PCT WO 98/09653). Ils ont aussi décrit dans des demandes non encore publiées d'autres dérivés d'amidines ou, plus récemment, des dérivés d'aminopyridines. Ces dérivés d'amidines ou d'aminopyridines présentent la particularité d'être à la fois des inhibiteurs de NO Synthases et des inhibiteurs de ROS.

La demande WO 95/05363 a pour objet des composés inhibiteurs de NO Synthases de formule générale **(A1)** 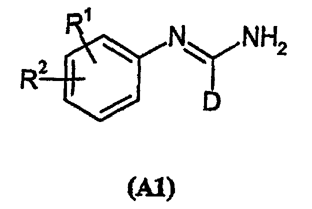 dans laquelle :
D représente phényle, pyridinyle ou un hétérocycle aromatique à 5 chaînons contenant de 1 à 4 hétéroatomes choisis parmi O, N et S, ces trois groupes étant éventuellement substitués par un ou des groupes choisis parmi (C₁-C₆)alkyle, (C₁-C₆)alkoxy, halogène, (C₁-C₆)perfluoroalkyIe, ou D représente (C₁-C₆)perfluoroalkyle ;
R¹ représente hydrogène, (C₁-C₆)alkyle ou halogène ;
R² représente -X(CH₂)ₙZCONR³R⁴, -X(CH₂)ₙNHCO(CH₂)ₛNR³R⁴, -X(CH₂)ₚNR³R⁴, -X(CH₂)ₙNHCOR⁵ ou -(CH₂)_{q}NHC(NH)R⁶,
R³ et R⁴ représentent indépendamment hydrogène, (C₁-C₆)alkyle, -(CH₂)ᵣ-A, -(CH₂)ₘOA, ou -CH(CH₃)(CH₂)ₜA ;
ou l'ensemble NR³R⁴ représente 1-indanyle, pipéronylamino, pipéridynyle, morpholinyle, pyrrolidinyle, 1,2,3,4-tétrahydroisoquinolinyle ou pipérazinyle éventuellement substitué en position 4 par (C₁-C₆)alkyle ;
R⁵ représente (C₁-C₆)alkyle, (C₁-C₆)perfluoroalkyle, -(CH₂)ᵣ-A ou -O(CH₂)_{w}A ;
A représente phényle, pyridinyle, pyrimidinyle ou hétérocycle aromatique à 5 chaînons contenant de 1 à 4 hétéroatomes choisis parmi O, N et S, ces 4 groupes étant éventuellement substitués par un ou des groupes choisis parmi (C₁-C₆)alkyle, halogène, nitro, cyano et trifluorométhyle ;
R⁶ représente phényle, pyridinyle ou un hétérocycle aromatique à 5 chaînons contenant de 1 à 4 hétéroatomes choisis parmi O, N et S, ces trois groupes étant éventuellement substitués par un ou des groupes choisis parmi (C₁-C₆)alkyle, (C₁-C₆)alkoxy, halogène, (C₁-C₆)perfluoroalkyle, ou R⁶ représente (C₁-C₆)perfluoroalkyle ;
n et r représentent indépendamment des entiers de 0 à 6 ;
p et w représentent indépendamment des entiers de 1 à 5 ;
m représente un entier de 2 à 5 ;
q et t représentent indépendamment des entiers de 0 à 5 ;
s représente un entier de 1 à 3 ;
X représente O ou une liaison ;
Z représente O, NR⁷ ou une liaison ;
R⁷ représente hydrogène ou (C₁-C₆)alkyle ;
étant entendu que :
(a) D, lorsque qu'il contient un hétéroatome, n'est pas relié au reste du composé de formule **(A1)** par l'hétéroatome ;
(b) lorsque R² représente -X(CH₂)ₙZCONR³R⁴ et ni X ni Z ne représentent une liaison, alors n représente un entier de 2 à 6;
(c) lorsque R² représente -X(CH₂)ₙNHCO(CH₂)ₛNR³R⁴ ou -X(CH₂)ₙNHCOR⁵ et X représente O, alors n représente un entier de 2 à 6 ;
(d) lorsque R² représente -X(CH₂)ₚNR³R⁴ et X représente O, alors p représente un entier de 2 à 5 ;
(e) lorsque R² représente -(CH₂)_{q}NHC(NH)R⁶, R¹ représente hydrogène, D représente phényle et R⁶ représente phényle, alors q ne représente pas 0 ;
(f) lorsque R² représente -(CH₂)_{q}NHC(NH)R⁶, R¹ représente hydrogène, D et R⁶ représentent 2-chlorophényle, alors q ne représente pas 0 ;
(g) lorsque R² représente -(CH₂)_{q}NHC(NH)R⁶, R¹ représente hydrogène, D et R⁶ représentent 3-pyridinyle, alors q ne représente pas 0 ; et
(h) lorsque R² représente -(CH₂)_{q}NHC(NH)R⁶, R¹ représente hydrogène, D et R⁶ représentent 4-pyridinyle, alors q ne représente pas 0.

La demande de brevet PCT WO 98/42696 a pour objet des composés inhibiteurs de NO Synthases et piégeurs de ROS de formule générale **(A2)** 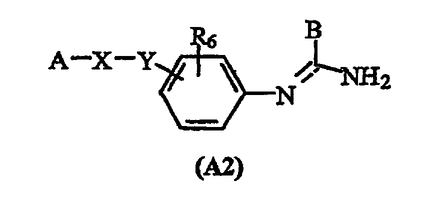 dans laquelle :
A représente l'un des radicaux 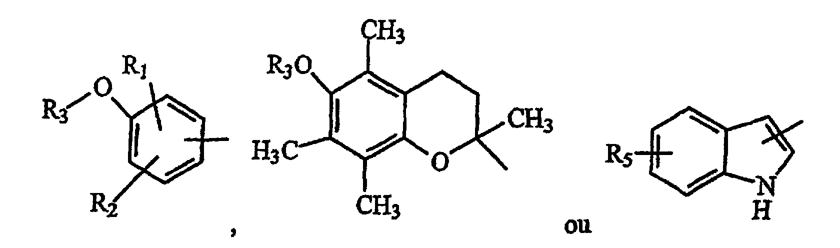 dans lesquels R₁ et R₂ représentent, indépendamment, un atome d'hydrogène, un halogène, le groupe OH, un radical allyle ou alkoxy linéaire ou ramifié ayant de 1 à 6 atomes de carbone,
   R₃ représente un atome d'hydrogène, un radical alkyle linéaire ou ramifié ayant de 1 à 6 atomes de carbone ou un radical -COR₄,
   R₄ représentant un radical alkyle linéaire ou ramifié ayant de 1 à 6 atomes de carbone,
   et R₅ représente un atome d'hydrogène, le groupe OH ou un radical alkyle ou alkoxy linéaire ou ramifié ayant de 1 à 6 atomes de carbone ;
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone;
X représente -Z₁-, -Z₁-CO-, -CH=CH-CO-, -Z₁-NR₃-CO-, -Z₁-NR₃-CS-, -Z₁-NR₃-SO₂- ou une simple liaison ;
Y représente un radical choisi parmi les radicaux -Z₂-Q, pipérazine, homopipérazine, 2-méthylpipérazine, 2,5-diméthyl-pipérazine, 4-aminopipéridine, -NR₃-Z₂-Q-, -NR₃-CO-Z₂-Q-, -NR₃-NH-CO-Z₂-, -NH-NH-Z₂-, -NR₃-O-Z₂-, -NR₃-SO₂-NR₃-Z₂-, -O-Z₂-Q-, -O-CO-Z₂-Q- ou S-Z₂-Q,
   dans lesquels Q représente une simple liaison, O-Z₃, R₃-N-Z₃ ou S-Z₃ ;
Z₁, Z₂ et Z₃ représentent indépendamment une simple liaison ou un radical alkylène linéaire ou ramifié ayant de 1 à 6 atomes de carbone;
et R₆ représente un atome d'hydrogène ou un groupe OH.

La présente invention a pour objet de nouveaux dérivés d'amidines, leur préparation et leur application en thérapeutique.

Les composés de l'invention répondent à la formule générale **(I')**: 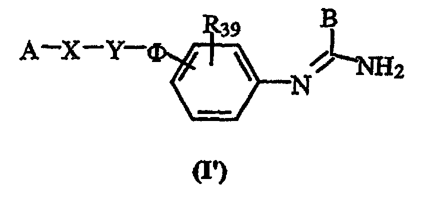 dans laquelle
A représente un radical 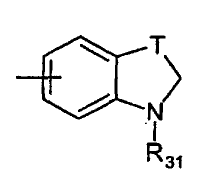 dans lequel T représente un radical -(CH₂)ₖ-, k représentant 1 ou 2, et R₃₁ représente un radical alkyle linéaire ou ramifié ayant de 1 à 6 atomes de carbone, ou l'un des radicaux aminoalkyle, alkylaminoalkyle, dialkylaminoalkyle, arylalkyle, diarylalkyle ou *bis*-arylalkyle dont le radical alkyle est linéaire ou ramifié et compte de 1 à 6 atomes de carbone ;
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
   ou encore B représente un radical NR₃₄R₃₅, dans lequel R₃₄ et R₃₅ représentant, indépendamment, un atome d'hydrogène ou un radical alkyle linéaire ou ramifié ayant de 1 à 6 atomes de carbone, ou R₃₄ et R₃₅ forment avec l'atome d'azote un hétérocycle non aromatique de cinq à six chaînons, les éléments de la chaîne étant choisis parmi un groupe composé de -CH₂-, -NH-, -O- ou -S- ;
X représente une liaison ou un radical -(CH₂)ₘ-, -(CH₂)ₘ-CO, -O-(CH₂)ₘ-, -S-(CH₂)ₘ-, -NR₃₆-(CH₂)ₘ,-, -CO-NR₃₆-, -O-(CH₂)ₘ-CO-, -S-(CH₂)ₘ-CO-, -NR₃₆-(CH₂)ₘ-CO-, -(CH₂)ₘ-C(OH)(CH₃)-CO-, -CH=CH- ou -CH=N- ;
Y représente une liaison ou un radical -(CH₂)ₙ- ou -(CH₂)ᵣ-Q-(CH₂)ₛ-,
   Q représentant un radical pipérazine, homopipérazine, 2-méthylpipérazine, 2,5-diméthylpipérazine, pipéridine, 1,2,3,6-tétrahydropyridine, pyrrolidine, azétidine, thiazolidine ou un cycle carboné saturé ayant de 3 à 7 chaînons ;
Φ représente une liaison ou un radical -(CH₂)ₚ-O-(CH₂)_{q}-, -(CH₂)ₚ-S-(CH₂)_{q}-, -(CH₂)ₚ-NR₃₇-(CH₂)_{q}-, -(CH₂)ₚ-CO-NR₃₇-(CH₂)_{q}- ou CO-(CH₂)ₚ-NR₃₇-(CH₂)_{q}-;
R₃₆ et R₃₇ représentent, indépendamment, un atome d'hydrogène, un radical allyle linéaire ou ramifié ayant de 1 à 6 atomes de carbone ou un radical -CO-R₃₈ dans lequel R₃₈ représente un radical alkyle ou alkoxy linéaire ou ramifié ayant de 1 à 6 atomes de carbone ;
R₃₉ représente un atome d'hydrogène ou un radical alkyle ou alkoxy linéaire ou ramifié ayant de 1 à 6 atomes de carbone ;
m, n, p, q, r et s étant des entiers de 0 à 6 ;
ou encore les sels des produits de formule générale **(I')**.

Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par aryle carbocyclique ou hétérocyclique, on entend un système carbocyclique ou hétérocyclique comprenant au moins un cycle aromatique, un système étant dit hétérocyclique lorsque l'un au moins des cycles qui le composent comporte un hétéroatome (O, N ou S). Par hétérocycle, on entend un système mono- ou polycyclique ledit système comprenant au moins un hétéroatome choisi parmi O, N et S et étant saturé, partiellement ou totalement insaturé ou aromatique. Par haloalkyle, on entend un radical alkyle dont au moins l'un des atomes d'hydrogène (et éventuellement tous) est remplacé par un atome halogène.

Par radicaux alkylthio, alkoxy, haloalkyle, haloalkoxy, aminoalkyle, alkényle, alkynyle, aralkyle et (hétérocyclo)alkyle, on entend respectivement les radicaux alkylthio, alkoxy, haloalkyle, haloalkoxy, aminoalkyle, alkényle, alkynyle, aralkyle et (hétérocyclo)alkyle dont le radical alkyle a la signification indiquée précédemment.

Par hétérocycle, on entend notamment les radicaux thiophène, pipéridine, pipérazine, quinoline, indoline et indole. Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

Parmi les radicaux A utilisables pour l'invention, on préférera en particulier les radicaux du type 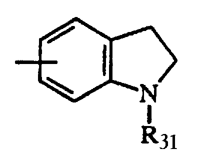 dans lequels R₃₁ représente un radical alkyle linéaire ou ramifié ayant de 1 à 6 atomes de carbone, aminoalkyle, alkylaminoalkyle, dialkylaminoalkyle, arylalkyle, diarylalkyle ou bis-arylalkyle, et notamment ceux dans lesquels R₃₁ représente le radical méthyle, benzyle ou naphtylméthyle.

De préférence, les composés selon l'invention seront l'un des composés suivants:
- 3-[(3-{[amino(2-thiényl)méthylidène]amino}-benzyl)amino]-N-(1-méthyl-2,3-dihydro-1H-indol-5-yl)propanamide ;
- 3-[(3-{[amino(2-thiényl)méthylidène]amino}-benzyl)amino]-N-(1-benzyl-2,3-dihydro-1H-indol-5-yl)propanamide;
- 3-[(3-{[amino(2-thiényl)méthylidène]amino}benzyl)amino]-N-[1-(1-naphthylméthyl)-2,3-dihydro-1H-indol-5-yl]propanamide ;
- 4-(4-{[amine(2-thiényl)méthylidène]amino}phényl)-N-{1-[3-(diméthylamino)propyl]-2,3-dihydro-1H-indol-5-yl}butanamide ;
- 3-[(5-{[amino(2-thiényl)méthylidène]amino}-2-méthoxybenzyl)amino]-N-[1-(1-naphthylméthyl)-2,3-dihydro-1H-indol-5-yl]propanamide ;
ou les sels de ces derniers.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention concerne de plus les composés de formule générale **(IS')**, intermédiaires de synthèse des produits de formule générale **(I')** dans lesquels A est un radical indoline substitué tel que défini précédemment, X représente le radical -NR₃₆-CO-, Y représente une liaison et Φ représente un radical -(CH₂)ₚ-NR₃₇-(CH₂)_{q}-, 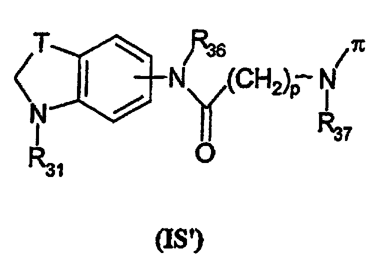 formule générale **(IS')** dans laquelle
π représente un atome d'hydrogène ou un groupe protecteur de type carbamate ;
R₃₆ et R₃₇ représentent, indépendamment, un atome d'hydrogéne, un radical alkyle linéaire ou ramifié ayant de 1 à 6 atomes de carbone ou un radical -CO-R₃₈ dans lequel R₃₈ représente un radical alkyle ou alkoxy linéaire ou ramifié ayant de 1 à 6 atomes de carbone;
T représente un radical -(CH₂)ₖ-, k représentant 1 ou 2 ;
R₃₁ représente un radical alkyle linéaire ou ramifié ayant de 1 à 6 atomes de carbone, ou arylalkyle, diarylalkyle, bis-arylalkyle, aminoalkyle, alkylaminoalkyle ou dialkylaminoalkyle, dont les radicaux alkyles sont linéaires ou ramifiés et comptent de 1 à 6 atomes de carbone ;
et p est un entier de 0 à 6.

L'invention a également pour objet, à titre de médicaments, les composés de formule générale **(I')** décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à inhiber la NO synthase neuronale ou la NO synthase inductible, à inhiber la péroxidation lipidique ou à assurer la double fonction d'inhibition de la NO synthase et d'inhibition de la péroxidation lipidique.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci.* **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

### PREPARATION DES COMPOSES DE L'INVENTION :

Les produits de formule générale **(I')** sont préparés comme décrit ci-après.

### Préparation des composés de formule générale (I') :

Les composés de formule générale **(I')** peuvent être préparés à partir des intermédiaires de formule générale **(II)** selon le schéma 1 où A, B, X, Y, Φ et R₃₉ sont tels que définis ci-dessus et Gp est un groupe protecteur de type carbamate, par exemple le groupe t-butoxycarbonyle.

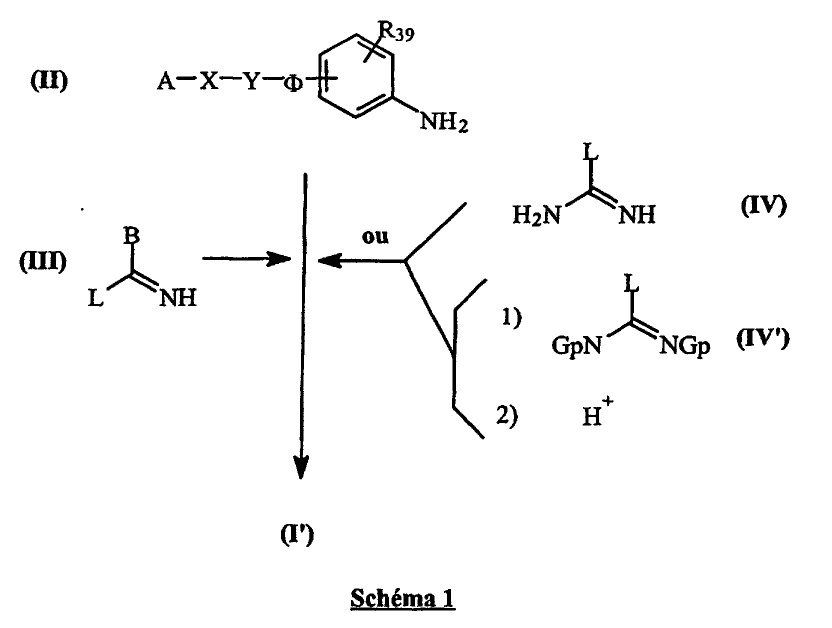

Les dérivés d'aniline de formule générale **(II)**, peuvent être condensés sur des composés de formule générale **(III)**, dans lesquels L représente un groupe partant (un radical alkoxy, alkylthio, aralkylthio, acide sulfonique, halogénure, alcool arylique ou tosyle), pour conduire aux composés finaux de formule générale **(I')** de type amidine substitué (cf. schéma 1). Par exemple, pour B = thiophène, on peut condenser les dérivés de formule générale **(II)** sur l'iodhydrat de S-méthylfhiophène thiocarboxamide, préparé selon une méthode de la littérature (*Ann. Chim.* (1962), **7**, 303-337). La condensation peut s'effectuer par chauffage dans un alcool (par exemple dans du méthanol ou de l'isopropanol), éventuellement en présence de DMF à une température de préférence comprise entre 50 et 100 °C pour une durée généralement comprise entre quelques heures et une nuit.

La salification des composés de formule générale **(I')**, lorsqu'elle est effectuée en présence d'un acide fort tel que, par exemple, l'acide chlorhydrique, permet de libérer simultanément les amines qui ont été protégées sous forme de carbamate de *tert*-butyle lors de la synthèse.

Dans les cas où B est une amine, les composés finaux de formule générale **(I')** sont des guanidines. Celles-ci peuvent être préparées, par exemple, par la condensation des amines de formule générale **(II)** avec les dérivés de formule générale **(IV)** ou **(IV')**. Les réactifs de formule générale **(IV)** dans lesquels L représente, par exemple, un cycle pyrazole sont condensés sur les amines de formule générale **(II)** selon les conditions décrites dans la littérature (*J. Org. Chem.* (1992) **57**, 2497-2502), de même que pour les réactifs de formule générate **(IV')** dans lesquels L représente, par exemple, un cycle pyrazole et Gp le groupement tBuOCO (*Tetrahedron Lett.* (1993) **34** (21), 3389-3392) ou bien lorsque L représente le groupement N-SO₂-CF₃ et Gp le groupement tBuOCO (*J. Org. Chem.* (1998) **63**, 3804-3805). Lors de l'ultime étape de la synthèse, la déprotection de la fonction guanidine est effectuée en présence d'un acide fort tel que par exemple l'acide trifluoroacétique.

L'invention a donc également pour objet un procédé de préparation de composés de formule générale **(I')** telle que définie ci-dessus, ledit procédé étant caractérisé en ce que l'on fait réagir un composé de formule générale **(II)** 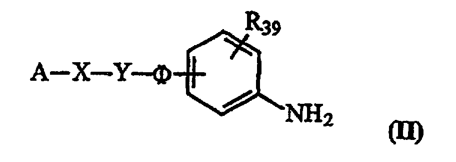 dans laquelle A, B, X, Y, Φ et R₃₉ sont tels que définis ci-dessus,
avec l'intermédiaire de formule générale **(III)** 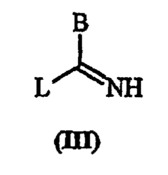 dans laquelle B est tel que défini ci-dessus et L représente un groupe partant, par exemple un radical alkoxy, alkylthio, aralkylthio, acide sulfonique, halogénure, alcool arylique ou tosyle.

L'invention a donc également pour objet un procédé de préparation de composés de formule générale **(I')** telle que définie ci-dessus et dans laquelle B représente une amine, ledit procédé étant caractérisé en ce que l'on fait réagir un composé de formule générale **(II)** 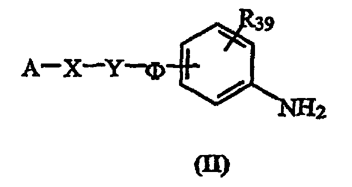 dans laquelle A, B, X, Y, Φ et R₃₉ sont tels que définis ci-dessus,
a) soit avec l'intermédiaire de formule générale **(IV)** 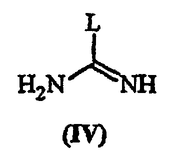 dans laquelle L représente un groupe partant, par exemple un radical alkoxy, alkylthio, aralkylthio, acide sulfonique, halogénure, alcool arylique ou tosyle,
b) soit avec l'intermédiaire de formule générale **(IV')** 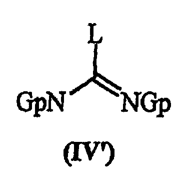 dans laquelle L représente un groupe partant, par exemple un radical alkoxy, alkylthio, aralkylthio, acide sulfonique, halogénure, alcool arylique ou tosyle, et Gp un groupe protecteur de type carbamate, par exemple le groupe t-butoxycarbonyle,
cette réaction étant suivie, dans le cas où l'on opte pour la réaction avec le composé de formule générale **(IV'),** par une hydrolyse en présence d'un acide fort, par exemple l'acide trifluoroacétique.

### Préparation des composés de formule générale (II) :

Les intermédiaires de formule générale **(II),** non commerciaux, sont obtenus soit à partir de la coupure d'un groupe protecteur, soit à partir de la réduction d'un précurseur de type nitro, comme illustrés dans les schémas synthétiques ci-dessous.

### Réduction des précurseurs de type nitro :

La réduction de la fonction nitro des intermédiaires de formule générale **(V)**, schéma 2, dans lesquels A, X, Y, Φ et R₃₉ sont tels que définis ci-dessus, est généralement effectuée par hydrogénation catalytique, dans l'éthanol, en présence de Pd/C, sauf dans les cas de molécules sensibles à ces conditions où le groupement nitro est sélectivement réduit, par exemple, en chauffant le produit dans un solvant approprié tel que l'acétate d'éthyle avec un peu d'éthanol en présence de SnCl₂ (*J. Heterocyclic Chem.* (1987), **24**, 927-930 ; *Tetrahedron Letters* (1984), **25**(8), 839-842) ou bien à l'aide de NaBH₄-BiCl₃ (*Synth. Com.* (1995) **25** (23), 3799-3803) dans un solvant tel que l'éthanol, ou alors en utilisant du Ni Raney additionné d'hydrate d'hydrazine (*Monatshefte für Chemie,* (1995), **126**,725-732), ou encore à l'aide de SnCl₂ en présence de Zn (*Synthesis* (1996), (9), 1076-1078).

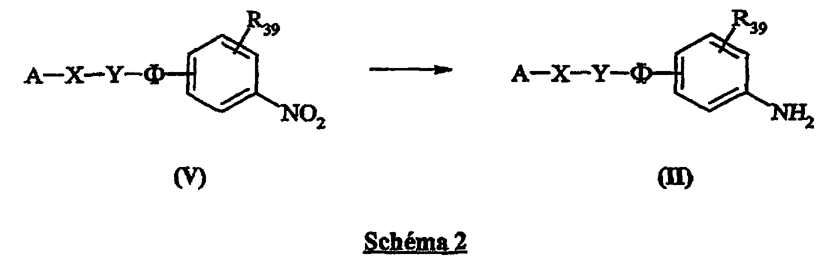

Dans le cas particulier des dérivés indoliques de formule générale **(V)** (schéma 6), les conditions de réduction douces de la fonction nitro (Zn dans l'acide acétique) s'accompagnent inévitablement de la perte de l'insaturation au niveau de la 1,2,3,6-tétrahydropyridine pour conduire à la pipéridine.

### Déprotection du groupe amino :

Les intermédiaires de formule générale **(II)**, dans lesquels A, X, Y, Φ et R₃₉ sont tels que définis ci-dessus, peuvent également être préparés à partir des intermédiaires de formule générale **(VI)**, schéma 3, qui sont des composés comportant une amine protégée sous forme, par exemple, de 2,5-diméthylpyrrole (N=Gp') ou de carbamate de *tert*-butyle (NH-Gp). Les pyrroles, par exemple, sont déprotégés par chauffage en présence de chlorhydrate d'hydroxylamine, pendant au moins 24 heures pour conduire finalement aux amines primaires de formule générale **(II)**. Les amines protégées sous forme de carbamates de *tert*-butyle sont libérées classiquement en milieu acide par traitement par l'acide trifluoroacétique ou l'acide chlorhydrique.

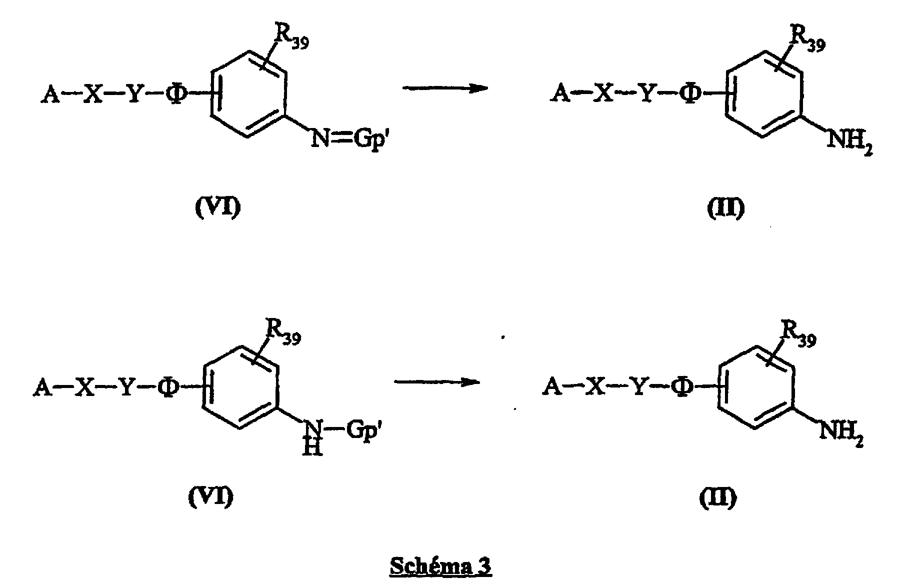

### Préparation des composés de formule générale (V) :

### Synthèse des carboxamides :

Les carboxamides de formule générale **(V)**, schéma 4, dans lesquels X représente -CO-NR₃₆- et A, Y, Q, Φ, R₃₉ et R₃₆ sont tels que définis ci-dessus, sont préparés par condensation des acides de formule générale **(VII)** avec les animes commerciales de formule générale **(VIII)** dans les conditions classiques de la synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) (*J. Med. Client* (1992), **35** (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)).
Les synthèses des acides de formule générale **(VII)** non commerciaux sont décrites au chapitre Préparation des Intermédiaires.

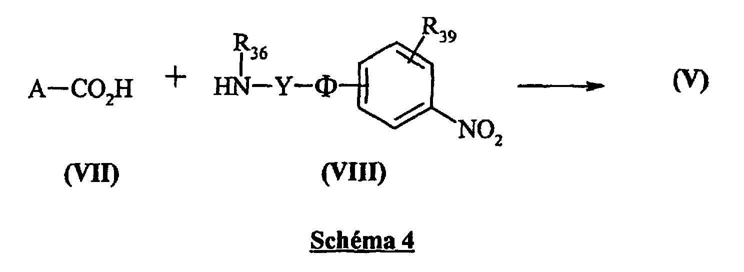

### (Série A-X-CO-N)

Les carboxamides de formule générale **(V)**, schéma 5, dans lesquels X représente -O-(CH₂)ₘ-, -S-(CH₂)ₘ- ou -NR₃₆-(CH₂)ₘ- avec A, Y, Φ, R₃₉ et R₃₆ tels que définis ci-dessus (Q étant un hétérocycle), sont préparés par condensation des acides de formule générale **(IX)** avec les amines de formule générale **(X)** ou **(XXII)** dans les conditions classiques de la synthèse peptidique comme précédemment décrit. Les synthèses des acides de formule générale **(IX)** et des amines de formule générale **(X)**, non commerciaux, sont décrites au chapitre Préparation des Intermédiaires.

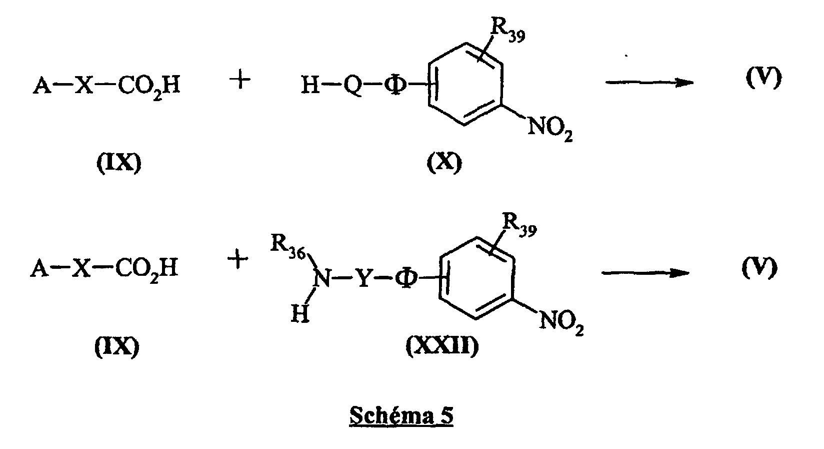

### Synthèse des dérivés de la 1,2,3,6-tétrahydropyridine :

Les dérivés indoliques de formule générale **(V')**, dans lesquels Q représente la 1,2,3,6-tétrahydropyridine, Φ, R₃₀ et R₃₉ étant tels que définis ci-dessus, sont préparés à partir des indoles substitués commerciaux de formule générale **(XI),** schéma 6. Le protocole expérimentale de la réaction de condensation sur la pipéridone est décrit dans *Eur. J. Med. Chem.* (1987) **22**, 33-43. L'intermédiaire de formule générale **(XII)** est ensuite classiquement condensé sur les dérivés halogénés de formule générale **(XIII)** en présence d'une base telle que, par exemple, Na₂CO₃, dans un solvant polaire approprié comme par exemple le DMF, pour conduire aux intermédiaires de formule générale **(V')** (cas particulier des intermédiaires de formule générale **(V)**).

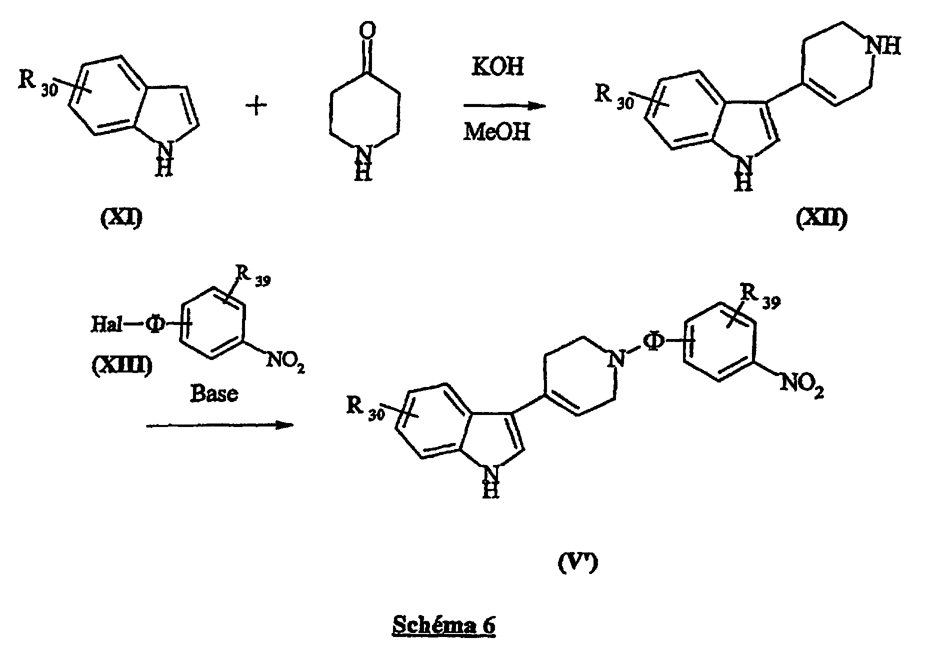

Les dérivés de formule générale **(Vbis)** (cas particulier des intermédiaires de formule générale **(V)**), schéma 7, dans lesquels Q représente la 1,2,3,6-tétrahydropyridine, Φ, Y et R₃₉ étant tels que définis ci-dessus, Hal étant un atome halogène, sont également préparés par condensation de la 4-phényl-1,2,3,6-tétrahydropyrine sur les dérivés halogènes de formule générale **(XIII)** en présence d'une base telle que, par exemple, K₂CO₃, dans un solvant polaire approprié tel que, par exemple, le DMF. Alternativement, les composés de formule générale **(Vbis)**, sont accessibles par condensation de type Mitsunobu (*Synthesis* (**1981**), 1) entre la 4-phényl-1,2,3,6-tétrahydropyrine et les dérivés alcooliques de formule générale **(XVIII)**.

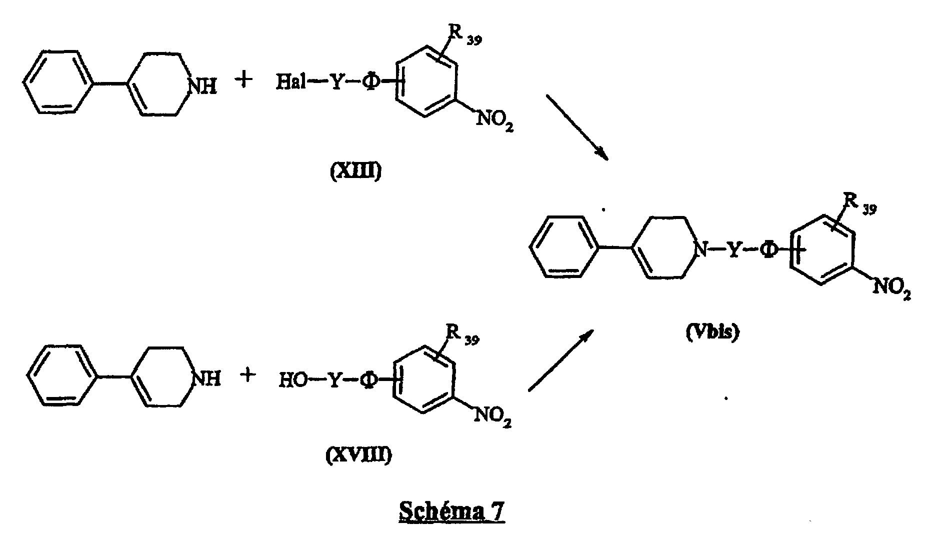

### Synthèse des éthers de formule générale (V) :

Lorsque Φ représente -(CH₂)ₚ-O-(CH₂)_{q}-, avec A, Q, R₃₉, p et q tels que définis ci-dessus, les éthers de formule générale **(V)**, schéma 9, peuvent être préparés en une seule étape par condensation des alcools de formule générale **(XV)** sur des dérivés halogénés de formule générale **(XVI)** en présence d'une base telle que par exemple NaH dans un solvant polaire tel que par exemple le THF.

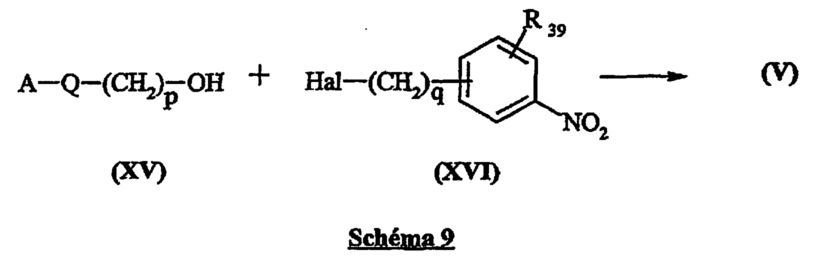

Lorsque Φ représente -(CH₂)ₚ-O-(CH₂)_{q}-, avec A, R₃₉, p et q tels que définis ci-dessus, les éthers de formule générale **(V)** peuvent également être préparés à partir des dérivés halogènes de formule **(XVII)** et des alcools de formule générale **(XVIII)**, schéma 10, en présence d'une base telle que par exemple NaH dans un solvant polaire tel que par exemple le DMF.

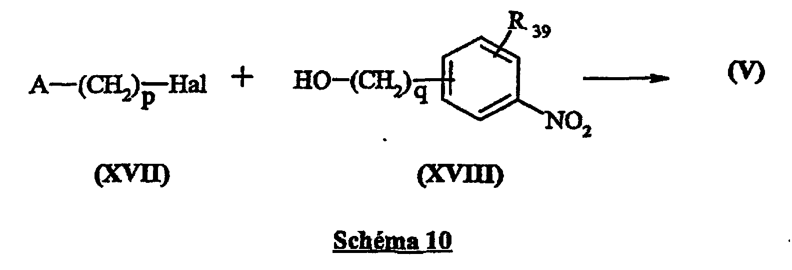

### Synthèse des carboxamides (série A-X-N-CO-) :

Les carboxamides de formule générale **(V)**, schéma 11, dans lesquels A, R₃₉, R₃₆, Y et **Φ** sont tels que définis ci-dessus, sont préparés dans les mêmes conditions de couplage peptidique que les carboxamides de la série A-X-CO-N-. Les préparations des amines de formule générale **(XIX)** et des acides de formule générale **(XX)** non commerciaux sont décrites au chapitre Préparation des Intermédiaires.

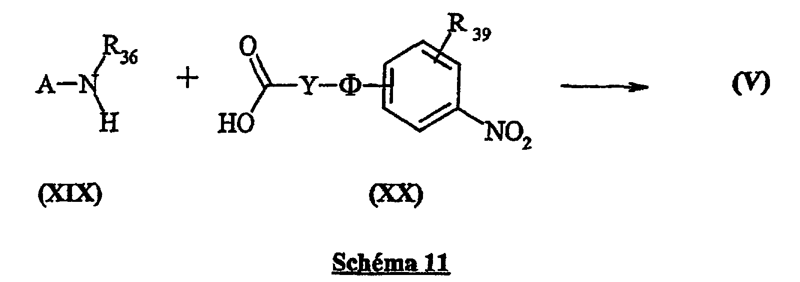

### Synthèse des amines par animation réductrice :

Les amines de formule générale **(V)**, schéma 12, dans lesquelles A, R₃₉, R_{36,} Y, m et Φ sont tels que définis ci-dessus, peuvent être préparées par condensation d'un aldéhyde de formule générale **(XXI)** avec une amine commerciale de formule générale **(XXII)** en milieu réducteur. La réaction a lieu dans un solvant alcoolique tel que, par exemple, le méthanol en présence de tamis moléculaire 4 Å pulvérulent, préalablement activé, et d'un agent réducteur tel que, par exemple, NaBH₄ ou NaBH₃CN. Avant l'addition du réducteur, certaines imines peuvent être isolées en tant qu'intermédiaire de formule générale **(V)**.

Les amines secondaires de formule générale **(V)** sont ensuite protégées sous forme de carbamate de *tert*-butyle en présence de di-*tert*-butyldicarbonate, d'une base telle que, par exemple, la triéthylamine et dans un solvant tel que, par exemple, le dichlorométhane. Les synthèses des aldéhydes de formule générale **(XXI)**, non commerciaux, sont décrites au chapitre Préparation des Intermédiaires.

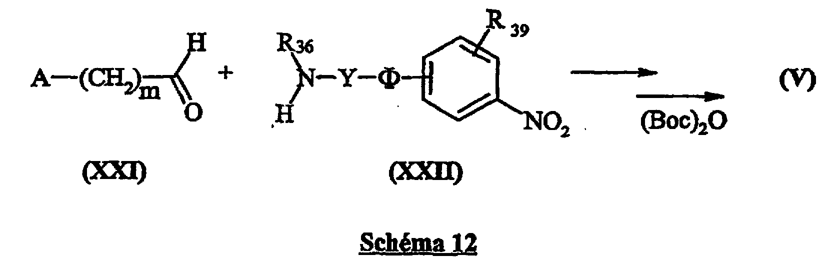

Les amines de formule générale **(V)**, schéma 13, dans lesquelles A, X, R₃₉, R₃₇, p et q sont tels que définis ci-dessus, peuvent également être préparées par condensation d'une amine de formule générale **(XXIII)** avec un aldéhyde commercial de formule générale **(XXIV)** en milieu réducteur dans les conditions précédemment décrites. Les amines secondaires de formule générale **(V)** sont ensuite protégées sous forme de carbamate de *tert*-butyle, dans les conditions précédemment décrites. Les synthèses des amines de formule générale **(XXIII)** sont décrites au chapitre Préparation des Intermédiaires. Par ailleurs, les aldéhydes non commerciaux de formule générale **(XXVI)** peuvent être préparés selon *J. Org. Chem.*, 1993, **58**, 1385-92.

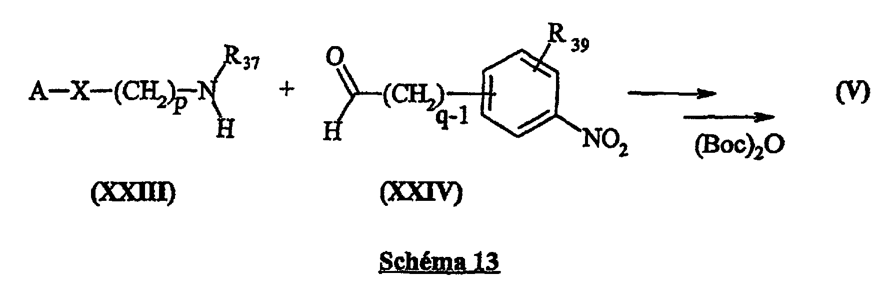

### Synthèse des amines par réduction des carboxamides :

Les amines de formule générale **(V)**, schéma 14, dans lesquels A, X, R₃₉, R₃₇ et q sont tels que définis ci-dessus, sont accessibles par réduction des dérivés carboxamides de formule générale **(V)** dont la synthèse est décrite au chapitre Synthèse des carboxamides (schéma 4). L'étape de réduction est effectuée en milieu anhydre, par chauffage à 70-80 °C, en présence d'un réactif sélectif des carboxamides tel que, par exemple, le BH₃.THF, dans un solvant tel que, par exemple, le THF. Les amines secondaires ainsi préparées peuvent être protégées sous forme de carbamate de *tert*-butyle dans les conditions précédemment décrites.

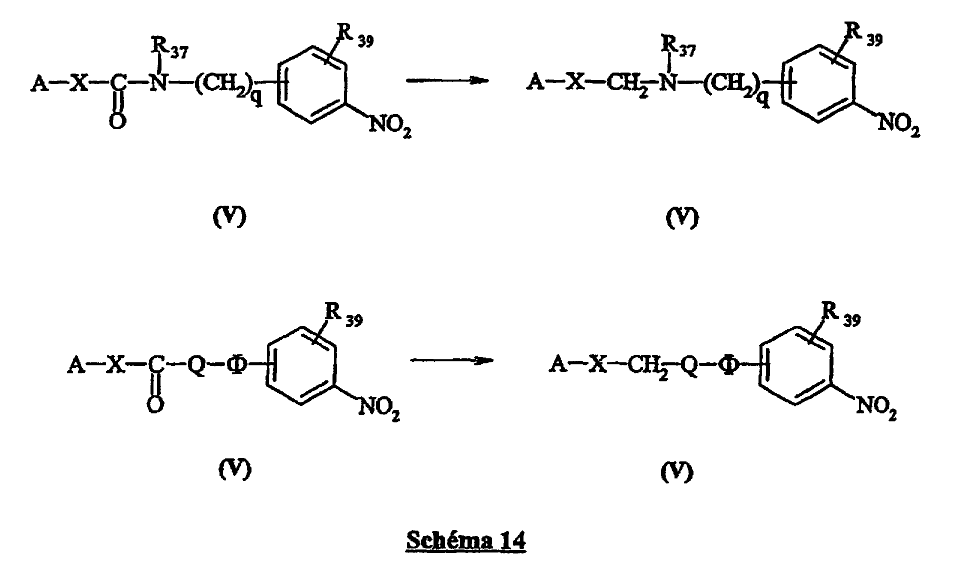

### Synthèse des urées :

Les urées de formule générale **(V)**, schéma 15, dans lesquels A, Y, Φ, R₃₉, R₃₆ et R₃₇ sont tels que définis ci-dessus, sont accessibles par réaction des amines de formule générale **(XIX)** avec les amines de formule générale **(XXII)** en présence de triphosgène et d'une base telle que, par exemple, la diisopropyléthylamine dans un solvant inerte tel que le dichlorométhane selon un protocole expérimental décrit dans *J. Org. Chem.* (1994) **59** (7), 1937-1938.

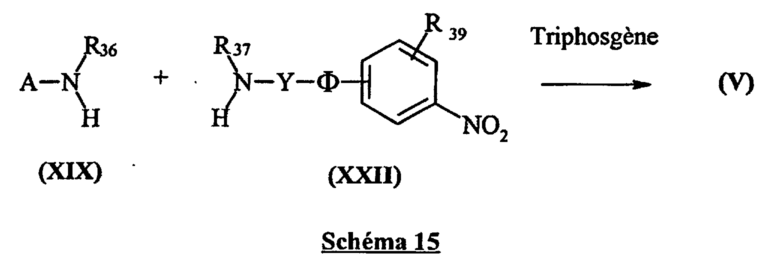

### Synthèse des carboxamides (série A-X-CO-N) :

Les carboxamides de formule générale **(VI)**, schéma 17, dans lesquels A, Y, Φ, R₃₉, R₃₆, Gp et Gp' sont tels que définis ci-dessus, peuvent être préparés à partir des acides commerciaux de formule générale **(VII)** et des amines de formule générale **(XXV)** ou **(XXVI)** dans les conditions classiques de la synthèse peptidique comme précédemment décrites. Les synthèses des amines de formule générale **(XXV)** et **(XXVI)** sont décrites au chapitre Préparation des Intermédiaires.

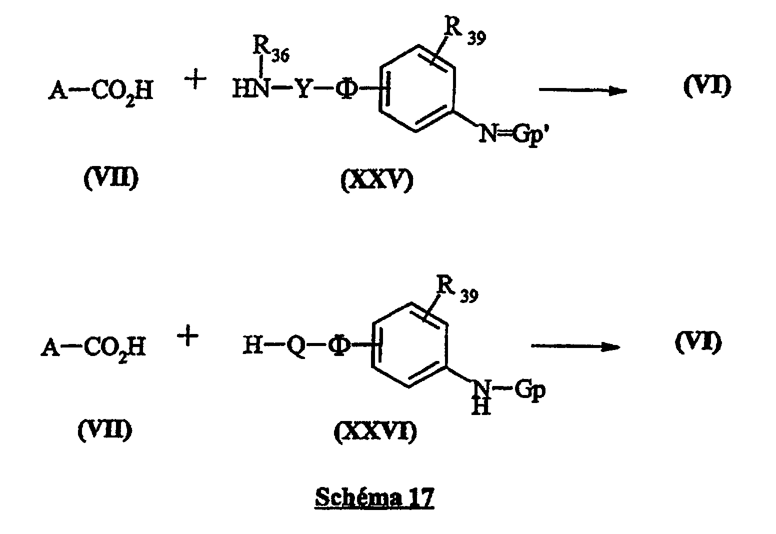

### Synthèse des carboxamides (série A-X-N-CO) :

Les carboxamides de formule générale **(VI)**, schéma 18, dans lesquels A, Y, Φ, R₃₉, R₃₆ et Gp sont tels que définis ci-dessus, peuvent être préparés par condensation des amines de formule générale **(XIX)**, précédemment décrites, avec les acides de formule générale **(XXVII)** dans les conditions classiques de la synthèse peptidique précédemment décrites. Les acides de formule générale **(XXVII)** sont aisément accessibles par protection de la fonction aniline sous forme, par exemple, de carbamate de *tert*-butyle dans des conditions classiques.

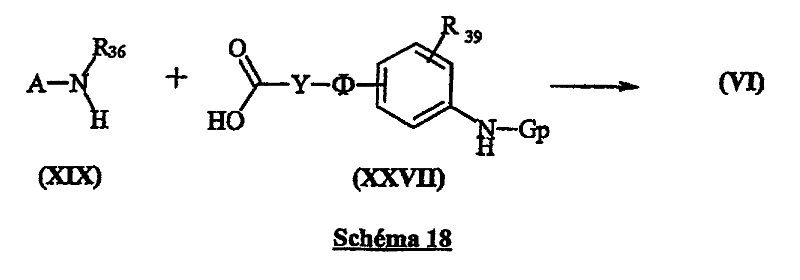

### Préparation des différents intermédiaires de synthèse :

### Synthèse des intermédiaires (VII) :

Les acides de formule générale **(VII)** non commerciaux dans lesquels A est tel que défini ci-dessus sont accessibles à partir de méthodes de la littérature. Par exemple, l'acide 2-hydroxy-4,5,6-triméthoxybenzoïque est obtenu en deux étapes à partir du 3,4,5-triméthoxyphénol selon *J. Org. Chem.* (1961) **26**, 1221-1223, *Acta Chem. Scandinavica* (1973) **27**, 888-890 ou *Can. J. Chem.* (1972) **50**, 1276-1282.

Certains acides de formule générale **(VII)**, dans lesquels A est tel que défini ci-dessus, comportent une amine (substituant R₁) qu'il est nécessaire de protéger sous forme de carbamate, en particulier de *tert*-*butyle*, avant d'effectuer l'étape de condensation. Cette protection s'effectue dans les conditions classiques décrites dans "M. Bodanszky et A. Bodanszky, *The Practice of Peptide Synthesis*, 145 (Springer Verlag, 1984)".

Les dérivés acides des benzofurannes sont préparés à partir d'un protocole expérimental décrit dans *J. Org. Chem.* (1989) **54**, 560-569.

### Synthèse des intermédiaires (IX) :

Les acides de formule générale **(IX)** non commerciaux dans lesquels X représente -O-(CH₂)ₘ- avec A tel que défini ci-dessus, sont préparés à partir des hydroquinones de formule générale **(IX.1)** obtenues selon la littérature (*J*. *Chem. Soc. Perkin 1* (1981) 303-306). La condensation sur des halogènoesters commerciaux de formule générale **(IX.2)** est effectuée en présence d'une base telle que, par exemple K₂CO₃, en chauffant dans un solvant polaire comme, par exemple, le THF pendant au moins 5 heures. Les esters de formule générale **(IX.3)** intermédiairement obtenus sont ensuite déprotégés (en milieu acide dans le cas des esters de *tert*-butyle) pour conduire aux acides de formule générale **(IX)**.

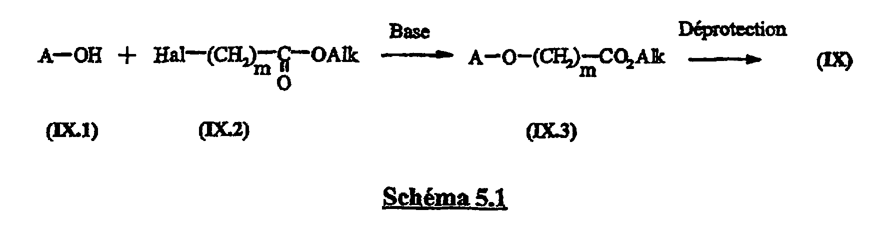

Les acides de formule générale **(IX)** dans lesquels X représente -S-(CH₂)ₘ- avec A tel que défini ci-dessus, sont préparés selon la littérature (*J. Med. Chem.* (1997) **40** (12), 1906-1918).

### Synthèse des intermédiaires (X) :

Les amines de formule générale **(X)** non commerciales, dans lesquelles Q représente homopipérazine, 2-méthylpipérazine, 2,5-diméthylpipérazine, 4-aminopipéridine, sont synthétisées en trois étapes à partir des diamines commerciales correspondantes. Les diamines sont sélectivement mono protégées sous forme de carbamate (*Synthesis* (1984), (12), 1032-1033; *Synth*. *Commun.* (1990), **20**, (16), 2559-2564) avant de réagir par substitution nucléophile sur un halogènonitrobenzène, en particulier le 4-fluoronitrobenzène. Les amines, précédemment protégées, sont libérées à la dernière étape, selon des méthodes décrites dans la littérature (T.W. Greene et P.G.M. Wuts, Protective Groups in Organic Synthesis, Second edition (Wiley-Interscience, 1991)), pour conduire aux intermédiaires de formula générale **(X)**.

### Synthèse des intermédiaires (XIX) :

Les amines de formule générale **(XIX)**, dérivés d'indoline ou de 1,2,3,4-tétrahydroquinoline, schéma 11.1, dans lesquelles T et R₃₁ sont tels que définis ci-dessus, peuvent être préparées à partir des dérivés nitro correspondants de formule générale **(XIX.1)**. La 6-nitro-1,2,3,4-tétrahydroquinoline est décrit dans *Can. J. Chem.* (1952), **30**, 720-722. L'alkylation de l'amine est classiquement effectuée par une base forte telle que, par exemple, NaH, dans un solvant aprotique polaire tel que, par exemple, le DMF en présence d'un dérivé halogéné tel que, par exemple, MeI ou PhCH₂Br. Le dérivé nitro de formule générale **(XIX.2)** intermédiairement obtenu est ensuite réduit, par exemple, par le Nickel de Raney en présence d'hydrate d'hydrazine pour conduire aux anilines de formule générale **(XIX)**.

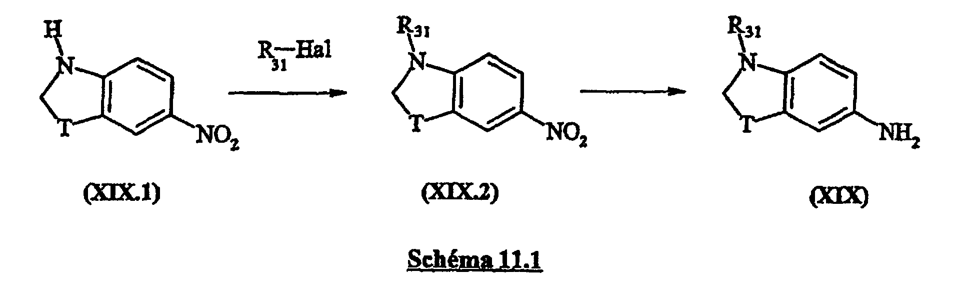

Egalement, certains dérivés de phénylènediamine de formule générale **(XIX)**, non commerciaux, peuvent être préparés selon *Farmaco* (1951) **6**, 713-717.

Dans le cas particulier où A est un dérivé phénolique, les anilines de formule générale **(XIX)** sont obtenues par hydrogénation, en présence de Pd/C, des dérivés nitrophénols précurseurs. Les dérivés nitrés des di-*tert*-butyl phénols sont accessibles selon une méthode décrite dans *J. Org. Chem.* (1968) **33** (1), 223-226.

### Synthèse des intermédiaires (XX) :

Les acides carboxyliques de formule générale **(XX)** non commerciaux dans lesquels R₃₉, Y et Φ sont tels que définis ci-dessus sont accessibles à partir de méthodes décrites dans la littérature. Les synthèses de plusieurs acides ω-(4'-nitrophényl)alkanoïques sont décrites dans *J. Med. Chem.* (1978) **21** (5), 430-437.

La synthèse des acides carboxyliques de formule générale **(XX)**, dans lesquels R₃₉ et Y sont tels que définis ci-dessus et Φ = thiazolidine, est réalisée à partir d'un protocole décrit dens *Liebigs Ann. Chem.* (1987), 927-934.

### Synthèse des intermédiaires (XXI) :

Les aldéhydes de formule générale **(XXI)** non commerciaux dans lesquels A et m sont tels que définis ci-dessus sont accessibles à partir de méthodes de la littérature : *Bull. Chem. Soc. Jpn.* (1978) **51** (8), 2433-2434, *Bioorg. Med. Chem. Lett.* (1998) **8**, 3453-3458.

### Synthèse des intermédiaires (XXIII) :

Les amines de formule générale **(XXIII)**, schéma 13.1, dans lesquelles A, X, R₃₇, Gp et p sont tels que définis ci-dessus, sont préparés par condensation des amines de formule générale **(XIX)**, précédemment décrites, et des aminoacides protégés de formule générale **(XXIII.1)**, dans les conditions classiques de la synthèse peptidique (voir chapitre Synthèse des carboxamides (schéma 4)). La déprotection de l'amine des composés de formule générale **(XXIII.2)** est ensuite effectuée en milieu acide tel que, par exemple, l'acide trifluoroacétique ou l'acide chlorhydrique.

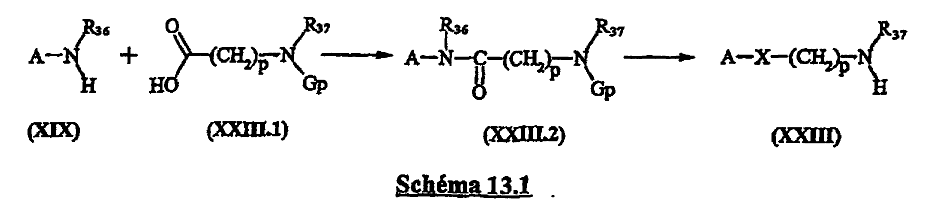

### Synthèse des intermédiaires (XXV) :

Les amines de formule générale **(XXV)**, dans lesquelles Y et **Φ** sont tels que définis ci-dessus, sont préparées en plusieurs étapes à partir de dérivés d'aniline commerciaux de formule générale **(XXV.1)**, schéma 17.1. Pour protéger l'aniline, il est nécessaire d'utiliser un groupe protecteur qui résiste à un milieu basique fort, par exemple le groupe 2,5-diméthylpyrrole. Par chauffage à reflux, dans un solvant approprié (p. ex. le toluène), d'un mélange de l'intermédiaire de formule générale **(XXV.1)** avec de la 2,5-hexanedione et de l'acide paratoluènesulfonique, avec élimination simultanée de l'eau formée au cours de la réaction, on obtient l'intermédiaire de formule générale **(XXV.2)**. L'intermédiaire de formule générale **(XXV.3)** est obtenu par la double alkylation d'un carbone à l'aide de deux équivalents d'une base forte, telle que, par exemple, NaH dans le DMSO (*J. Org. Chem.* (1971) **36** (9), 1308-1309), en présence d'un dérivé dihalogéné. Les amines de formule générale **(XXV)** sont ensuite obtenues par réduction du nitrile à l'aide, par exemple, de LiAlH₄, dans solvant tel que, par exemple, le THF anhydre.

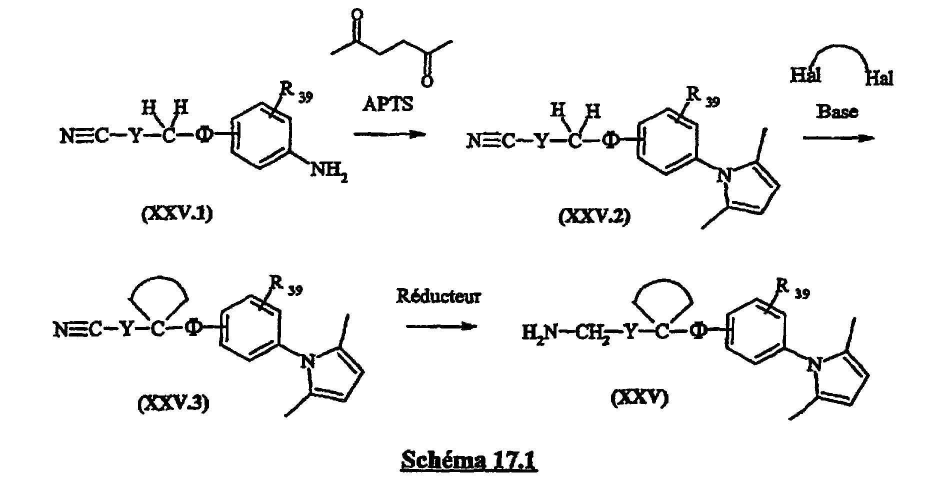

### Synthèse des intermédiaires (XXVI) :

Les amines de formule générale **(XXVI)**, schéma 17.2, dans lesquelles R₃₉ et Φ sont tels que définis ci-dessus, sont préparées en plusieurs étapes à partir d'un dérivé commercial d'azétidine et d'un dérivé halogéné de formule générale **(XIII)**. L'étape de condensation est effectuée classiquement en présence d'une base forte telle que, par exemple, NaH dans un solvant anhydre inerte tel que, par exemple, le THF. La réduction du dérivé nitro par le chlorure d'étain (*J. Heterocyclic Chem.* (1987), **24**, 927-930 ; *Tetrahedron Letters* (1984), **25** (8), 839-842) conduit au dérivé d'aniline de formule générale **(XXVI.2)** qui est directement protégé sous forme de carbamate de *tert*-butyle dans des conditions précédemment décrites. L'amine de formule générale **(XXVI)** est obtenue par hydrogènolyse du groupe dibenzyle en présence d'hydroxyde de palladium.

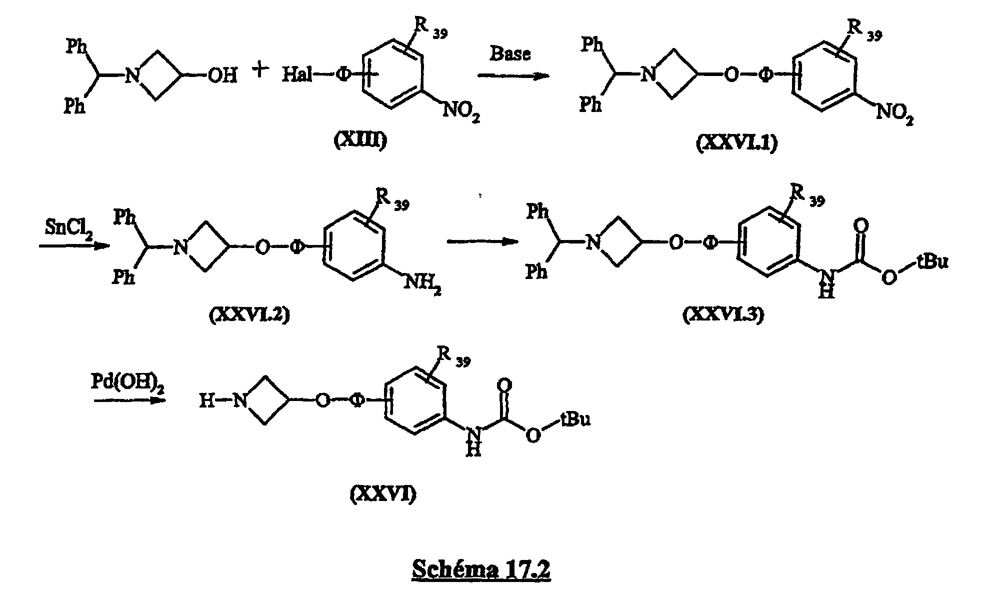

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES (seuls les exemples marqués par un * font partie de l'invention revendiquée)

### Exemple 1 : chlorhydrate de 2-hydroxy-5-méthoxy-N-{2-[4-[(2-thiényl(imino)méthyl)amino]phényl]éthyl}-benzamide:

### 1.1.) 2-hydroxy-5-méthoxy-N-{2-(4-nitrophényl)éthyl}-benzamide :

A une solution de 1,5 g (8,9 mmol) d'acide 5-méthoxy-salicylique dans du dichlorométhane (80 ml) on ajoute du chlorhydrate de 4-nitrophénétylamine (1,81 g ; 8,9 mmol), de la triéthylamine (2,8 ml ; 20 mmol), de l'hydroxybenzotriazole (1,45 g ; 10,7 mmol) et du chlorhydrate de 1-(3-diméthylaminopropyl)-3-éthyl-carbodiimide (3,75 g ; 19,62 mmol). Le milieu réactionnel est agité pendant une nuit à 25 °C. L'ensemble est dilué avec 40 ml d'eau et agité pendant dix minutes et le produit est extrait au dichlorométhane. La solution organique est séchée sur sulfate de sodium, filtrée et concentrée sous vide et le résidu d'évaporation est purifié sur colonne de silice (éluant = acétate d'éthyle/heptane ; 50/50) pour conduire à un solide blanc obtenu avec un rendement de 64%. Point de fusion : 200 °C.
RMN ¹H (DMSO d6, 400 MHz, δ) : 3,00 (m, 2H, CH₂) ; 3,60 (m, 2H, CH₂) ; 3,70 (S, 3H, -OCH₃) ; 6,80 (d, 1H, arom., J = 8,8 Hz) ; 7,00 (d, 1H, arom., J = 8,8 Hz) ; 7,35 (s, 1H, arom.) ; 7,50 (d, 2H, arom., J = 7,8 Hz) ; 8,10 (d, 2H, arom., J = 7,6 Hz) ; 8,90 (s large, 1H, CO-NH) ; 11,90 (s large, 1H, -OH).

### 1.2) 2-hydroxy-5-méthoxy-N-{2-(4-aminophényl)éthyl}-benzamide :

On dissout l'intermédiaire 1.1, 2,10 g (6,64 mmol) dans un mélange d'éthanol (40 ml) et de dichlorométhane (60 ml) et on ajoute 0,3 g de palladium sur charbon (10%). L'ensemble est placé sous atmosphère d'hydrogène sous 4 bars de pression. Le catalyseur est filtré et le solvant évaporé sous pression réduite. Le résidu d'évaporation est purifié sur colonne de silice (éluant = acétate d'éthyle/heptane ; 50/50) pour conduire à un solide blanc-crème. Point de fusion : 130 °C.
RMN ¹H (DMSO d6, 400 MHz, δ) : 2,67 (t, 2H, CH₂, J = 7,4 Hz) ; 3,43 (m, 2H, CH₂) ; 3.73 (S, 3H, -OCH₃) ; 4,90 (S, 2H, NH₂) ; 6,50 (d, 2H, arom., J = 7,8 Hz) ; 6,82 (d, 1H, arom., J = 8,8 Hz) ; 6,90 (d, 2H, arom., J = 7,8 Hz) ; 7,00 (d, 1H, arom., J = 8,8 Hz) ; 7,39 (S, 1H, arom.) ; 8,87 (s large, 1H, CO-NH) ; 12,09 (s large, 1H, -OH).

### 1.3) Chlorhydrate de 2-hydroxy-5-méthoxy-N-{2-[4-[(2-thiényl(imino)méthyl)amino] phényl]éthyl}-benzamide :

L'intermédiaire 1.2 (0,6 g ; 2,1 mmol) est dissout dans du 2-propanol (10 ml), on ajoute 0,896 g d'iodhydrate de S-méthyl-2-thiophènethiocarboximide (3,14 mmol) (Ann. Chim. (1962), 7, 303-337).

Après chauffage à 50 °C pendant 15 heures, le mélange réactionnel est concentré à sec sous vide. Le résidu est repris dans de l'acétate d'éthyle et une solution saturée de carbonate de sodium. Après décantation, la phase organique est lavée successivement par 50 ml d'une solution saturée de carbonate de sodium, d'eau et de saumure. La solution organique est séchée sur sulfate de sodium, filtrée et évaporée sous pression réduite. Le résidu d'évaporation est purifié sur colone de silice (éluant éthanol/dichlorométhane, 5/95). On obtient 0,523 g de base libre. On prépare le chlorohydrate à partir de 0,523 g (1,32 mmol) de base dissoute dans 20 ml d'acétone et salifiée en présence de 2,0 ml (2,0 mmol) d'une solution molaire de HCl dans l'éther diéthylique anhydre. Les cristaux obtenus sont filtrés et rincés à l'éther diéthylique pour obtenir après séchage 0,58 g (rendement de 64%) du produit recherché sous forme d'un solide blanc. Point de fusion : 242 °C.
RMN ¹H (DMSO d6, 400 MHz, δ) : 2,94 (t, 2H, CH₂, J = 7,2 Hz) ; 3,58 (m, 2H, CH₂) ; 3,73 (s, 3H, -OCH₃) ; 6,85 (d, 1H, arom., J = 9,0 Hz) ; 7,01 (d, 1H, arom., J = 8,8 Hz) ; 7,20 - 7,70 (m, 6H, arom.) ; 8,16 (m, 2H, thiophène) ; 8,88 (s large, 1H, NH⁺) ; 9,11 (t large, 1H, CO-NH, J = 5,15 Hz) ; 9,83 (s large, 1H, NH⁺) ; 11,54 (s large, 1H, NH⁺) ; 12,10 (s, 1H, -OH).
IR : ν_{C=N} (amidine) : 1645 cm-¹ ; ν_{C=O} (amide) : 1645 cm⁻¹.

### Exemple 14 : chlorhydrate de N-(2-hydroxy-3-tert-butyl-5-méthoxy)-4-{[2-thiényl(imino)méthyl]amino}benzène-butanamide

### 14.1.) N-(2-méthoxy-méthoxy-3-tert-butyl-5-méthoxy)-4-nitrobenzène-butanamide

Le protocole expérimental utilisé est le même que celui décrit pour l'intermédiaire 1.1, l'acide 4-(4-nitrophényl)butanoïque remplaçant l'acide 5-méthoxysalicylique et la 2-méthoxyméthoxy-3-*tert*-butyl-5-méthoxyaniline (*Biorg & Med Chem.* (1998), **6**, 849-868) remplaçant le chlorohydrate de 4-nitrophénétylamine. On obtient une huile rouge avec un rendement de 82%.
RMN ¹H (DMSO d6, 400 MHz, δ) : 1,32 (s, 9H, t-Butyl) ; 1,93 (m, 2H, CH₂) ; 2,37 (m, 2H, CH₂) ; 2,77 (m, 2H, CH₂) ; 3,53 (s, 3H, OCH₃) ; 3,68 (s, 3H, OCH₃) ; 4,90 (s, 2H, -OCH₂O-) ; 6,57 (s, 1H, arom.) ; 7,38 (s, 1H, arom.) ; 7,51 (d, 2H, arom., J = 8,50 Hz) ; 8,16 (d, 2H, arom., J = 8,50 Hz) ; 9,28 (s, 1H, NHCO).

### 14.2) N-(2-hydroxy-3-tert-butyl-5-méthoxy)-4-nitro-benzènebutanamide

A une solution de 1,49 g (3,86 mmol) de N-(2-méthoxy-méthoxy-3-tert-butyl-5-méthoxy)4-nitrobenzène-butanamide dans du méthanol (30 ml) on ajoute de l'acide chlorohydrique concentré (0,6 ml). Le milieu réactionnel est agité pendant une nuit à 25 °C. Les solvants sont évaporés et le résidu lavé successivement par 3 x 40 ml d'eau puis 40 ml de saumure. La solution organique est séchée sur sulfate de sodium, filtrée et concentrée sous pression réduite pour donner une huile brune, suffisamment pure pour être utilisée directement dans l'étape suivante.
RMN ¹H (DMSO d6, 400 MHz, δ) : 1,34 (s, 9H, t-Bu) ; 1,76 (m, 2H, CH₂) ; 2,44 (m, 2H, CH₂) ; 2,78 (m, 2H, CH₂) ; 3,65 (s, 3H, OCH₃) ; 6,61 (s, 1H, arom.); 6,66 (S, 1H, arom.) ; 7,53 (d, 2H, arom, J = 8,62 Hz) ; 8,18 (d, 2H, arom., J = 8,62 Hz) ; 8,56 (s, 1H, NH-CO) ; 9,94 (s, 1H, -OH).

### 14.3) N-(2-hydroxy-3-tert-butyl-5-méthoxy)-4-aminobenzène-butanamide

Le protocole expérimental utilisé est le même que celui décrit pour l'intermédiaire 1.2, le N-(2-hydroxy-3-tert-butyl-5-méthoxy)-4-nitro-benzènebutanamide remplaçant le 2-hydroxy-5-méthoxy-N-{2-(4-nitrophényl)éthyl}-benzamide.

### 14.4) Chlorhydrate de N-(2-hydroxy-3-tert-butyl-5-méthoxy)-4-{[2-thiényl(imino) méthyl]amino}benzènebutanamide.

Le protocole expérimental utilisé est le même que celui décrit pour l'exemple 3, le N-(2-hydroxy-3-tert-butyl-5-méthoxy)-4-aminobenzène-butanamide remplaçant le 2-hydroxy-5-méthoxy-N-{2-(4-aminophényl)éthyl}-benzamide. Point de fusion : 146-154 °C.
RMN ¹H (DMSO d6, 400 MHz, δ) : 1,34 (s, 9H, t-Butyl) ; 1,96 (m, 2H, CH₂) ; 2,70 (m, 2H, CH₂) ; 3,42 (m, 2H, CH₂) ; 3,65 (s, 3H, -OCH₃) ; 6,59 (s, 1H, arom.) ; 6,83 (s, 1H, arom.) ; 7,36-7,41 (m, 5H, arom.) ; 8,17 (m, 2H, thiophène) ; 8,75 (s, 1H, NHCO) ; 8,90 (s large, 1H, NH⁺) ; 9,80 (s large, 1H, NH⁺); 10,35 (s, 1H, -OH) ; 11,60 (s large, 1H, NH⁺).
IR : ν_{C=N} (amidine) : 1596 cm⁻¹ ; ν_{C=O} (amide) : 1698 cm⁻¹.

### Exemple 17 : iodhydrate de N-(4-hydroxyphényl)-2-thiophènecarboximidamide :

Un mélange composé de 0,55 g (5 mmol) de 4-aminophénol et de 1,42 g (5 mmol) de iodhydrate de S-méthyl-2-thiophènethiocarboximide en solution dans 20 ml d'isopropanol est agité pendant 2 heures à 40 °C. Le précipité formé est alors filtré et rincé par 2 fois 25 ml d'éther éthylique. Après séchage on obtient le produit attendu sous forme d'une poudre blanche avec un rendement de 77%.
Point de fusion : 258-259 °C.

### Exemple 18 : chlorhydrate de N-(2-hydroxyphényl)-2-thiophènecarboximidamide :

Le protocole expérimental utilisé est le même que celui décrit pour l'exemple 17, le 2-aminophénol remplaçant le 4-aminophénol. Le produit de la réaction est purifié sur colonne de silice (éluant : dichlorométhane / méthanol : 9/1). La base libre est alors salifiée à l'aide d'une solution 1M de HCl dans l'éther éthylique anhydre. On obtient le chlorhydrate sous forme d'une poudre crème avec un rendement de 20%.
Point de fusion : 206-207 °C.

### Exemple 19 : iodhydrate de N-(3-hydroxyphényl)-2-thiophènecarboximidamide:

Le protocole expérimental utilisé est le même que celui décrit pour l'exemple 17, le 3-aminophénol remplaçant le 4-aminophénol. Après séchage on obtient le produit attendu sous forme d'une poudre crème avec un rendement de 72%.
Point de fusion : 198-200 °C.

### Exemple 20 : chlorhydrate de N-(3-hydroxy-4-méthoxyphényl)-2-thiophènecarboximidamide :

Le protocole expérimental utilisé est le même que celui décrit pour l'exemple 18, le 5-amino-2-méthoxyphénol remplaçant le 2-aminophénol. Après séchage on obtient le produit attendu sous forme d'une poudre crème avec un rendement de 30%.
Point de fusion : 253-254 °C.

### Exemple 21 : chlorhydrate de N-(3-hydroxy-4-méthylphényl)-2-thiophènecarboximidamide:

Le protocole expérimental utilisé est le même que celui décrit pour l'exemple 18, le 3-amino-o-crésol remplaçant le 2-aminophénol. Après séchage on obtient le produit attendu sous forme d'une poudre crème avec un rendement de 61%.
Point de fusion : 245-246 °C.

### Exemple 22 : chlorhydrate de N-(4-méthoxyphényl)-2-thiophènecarboximidamide:

Le protocole expérimental utilisé est le même que celui décrit pour l'exemple 18, la p-anisidine remplaçant le 2-aminophénol. Après séchage on obtient le produit attendu sous forme d'une poudre crème avec un rendement de 40%.
Point de fusion 78-79 °C.

### Exemple 23 : iodhydrate de N-(3,5-diméthyl-4-hydroxyphényl)-2-thiophènecarboximidamide :

### 23.1) 4-amino-2,6-diméthylphénol :

Une solution de 1 g (6 mmol) de 2,6-diméthyl-4-nitrophénol dans 20 ml d'éthanol est placée sous 1,5 bar d'hydrogène en présence de Pd/C à 10% pendant 1 heure. Le Pd/C est éliminé par filtration sur célite et le filtrat est concentré sous pression réduite. Le résidu d'évaporation cristallise spontanément, il est lavé par 2 fois 50 ml d'heptane et séché une nuit sous vide. On obtient un solide couleur crème avec un rendement de 86%.
Point de fusion : 139-140 °C.

### 23.2) Iodhydrate de N-(3,5-diméthyl-4-hydroxyphényl)-2-thiophènecarboximidamide

Le protocole expérimental utilisé est le même que celui décrit pour l'exemple 17, le 4-amino-2,6-diméthylphénol remplaçant le 4-aminophénol. Après séchage on obtient le produit attendu sous forme d'une poudre blanche avec un rendement de 65%.
Point de fusion : 253-254 °C.

### Exemple 24 : Chlorhydrate de N-(3,5-dichloro-4-hydroxyphényl)-2-thiophènecarboximidamide :

Le protocole expérimental utilisé est le même que celui décrit pour l'exemple 18, le 4-amine-2,6-dichlorophénol remplaçant le 2-aminophénol. Après séchage on obtient le produit attendu sous forme d'une poudre crème avec un rendement de 30%.
Point de fusion : >260°C.

### Exemple 25 : chlorhydrate de N-(3,5-bis-(1,1-diméthyléthyl)-4-hydroxyphényl)-2-thiophènecarboximidamide :

### 25.1) 4-amino-2,6-bis-(1,1-diméthyléthyl)-phénol :

Dans une bouteille de Parr de 250 ml, 3,6 g (14 mmol) de 4-nitro-2,6-bis-(1,1-diméthyléthyl)-phénol (*J. Org. Chem.* (1968), **33** (1), 223-226) sont dissous dans 60 ml d'un mélange (2/1) d'éthanol et dichlorométhane en présence d'une quantité catalytique de Pd/C à 10 %. Le mélange est agité pendant 2 heures, à 20 °C, sous 20 PSI d'hydrogène. Après filtration sur célite, le filtrat est concentré à sec sous pression réduite. La poudre rousse obtenue est suspendue dans de l'heptane (30 ml), filtrée et rincée par le même volume d'heptane. Le produit attendu est obtenu sous forme d'une poudre rose saumon avec un rendement de 50 % (1,56 g).
Point de fusion : 123-124 °C.
RMN ¹H (100 MHz, CDCl₃, δ) : 6,60 (s, 2H, Ph) ; 4,65 (s large, 1H, OH) ; 3,15 (s large, 2H, NH₂) ; 1,42 (s, 18H, 2x tBu).

### 25.2) Chlorhydrate de N-(3,5-bis-(1,1-diméthyléthyl)-4-hydroxyphényl)-2-thiophénecarboximidamide :

Le protocole expérimental utilisé est le même que celui décrit pour l'exemple 18, le 4-amino-2,6-bis-(1,1-diméthyléthyl)-phénol remplaçant le 2-aminophénol. Après séchage on obtient le produit attendu sous forme d'une poudre jaune pâle avec un rendement de 65%.
Point de fusion : 258-259 °C.
RMN¹H (400 MHz, DMSO d6, δ) : 1,42 (s, 18H, 2x tBu) ; 7,18 (s, 2H, Ph) ; 7,38 (s, 1H, OH) ; 7,39 (s, 1H, thiophène) ; 8,18 (m, 2H, thiophène) ; 8,80 (s large, 1H, NH+) ; 9,70 (s large, 1H, NH+) ; 11,30 (s large, 1H, NH+).

### Exemple 26 : N-{4-[4-[(3,4-dihydro-6-hydroxy-2,5,7,8-tétraméthyl-2H-1-benzopyran-2-yl)méthyl]-1-pipérazinyl]phényl}-2-thiophènecarboximidamide :

### 26.1) 3,4-dihydro-2,5,7,8-tétraméthyl-2-{4[(phénylméthyl)-1-pipérazinyl]-carbonyl}-2H-1-benzopyran-6-ol :

A une solution de 5 g (20 mmol) de trolox dans 40 ml de THF, on ajoute 3,3 g (20,4 mmol) de 1,1'-carbonyldiimidazole. Après 1 heure d'agitation à 23 °C, on ajoute 3,52 g (20 mmol) de 1-benzyl-pipérazine dissous dans 20 ml de THF. Après agitation pendant 15 heures, le mélange réactionnel est concentré sous vide. Le résidu d'évaporation est dissous dans 50 ml de dichlorométhane et la solution est lavée par 2 fois 50 ml d'eau. La phase organique est séchée sur sulfate de sodium, filtrée et concentrée sous vide. Le résidu d'évaporation est purifié sur une colonne de silice (éluant : acétate d'éthyle/heptane : 3/2). On obtient 6,17 g d'une poudre blanche avec un rendement de 75%.
Point de fusion : 63-65 °C.

### 26.2) 3,4-dihydro-2,5,7,8-tétraméthyl-2-{4[(phénylméthyl)-1-pipérazinyl]-méthyl}-2H-1-benzopyran-6-ol :

A une suspension de 0,74 g (19,6 mmol) de LiAlH₄ dans 20ml de THF à 0 °C, on ajoute goutte-à-goutte une solution de 4 g (9,8 mmol) de l'intermédiaire 26.1. Après 15 minutes à 0 °C, le mélange réactionnel est agité 18 heures à 23 °C. La réaction est ensuite refroidie à l'aide d'un bain de glace et hydrolysée par addition successive de 4ml d'acétate d'éthyle, 0,8 ml d'eau, 0,8 ml de soude à 15% et finalement 2,4 ml d'eau. Le mélange réactionnel est ensuite filtré sur célite et le précipité lavé par 3 fois 25 ml d'acétate d'éthyle. Le filtrat est concentré sous vide et le résidu est purifié sur une colonne de silice (éluant : acétate d'éthyle/heptane : 1/1). On obtient une huile orange avec un rendement de 65%.

### 26.3) 3,4-dihydro-2,5,7,8-tétraméthyl-2-[(1-pipérazinyl)-méthyl]-2H-1-benzopyran-6-ol:

On ajoute 0,4 g de Pd/C à 10% à une solution de 2,52 g (6,39 mmol) de l'intermédiaire 26.2 dans 15 ml d'acide acétique. L'ensemble est agité sous 3 bars d'hydrogène pendant 1 heure. Le catalyseur est éliminé par filtration et le filtrat est concentré sous vide. Le résidu est partitionné dans un mélange de 80ml d'acétate d'éthyle et 100ml de NaOH 2N. Après décantation, la solution organique est lavée par 2 fois 150 ml d'eau, séchée sur sulfate de sodium et filtrée. Le filtrat est concentré sous vide et le résidu purifié sur une colonne de silice (éluant : dichlorométhane/méthanol/NH₄OH (28%) : 25/1/0,5). On obtient un solide blanc avec un rendement de 62%.

### 26.4) 3,4-dihydro-2,5,7,8-tétraméthyl-2-{4[(4-nitrophényl)-1-pipérazinyl]-méthyl}-2H-1-benzopyran-6-ol :

On dissout 0,6 g (1,97 mmol de l'intermédiaire 26.3, 0,54 g (3,94 mmol) de carbonate de potassium et 0,31 g (2,17 mmol) de 1-fluoro-4-nitrobenzène dans 20 ml de DMF. Le mélange réactionnel est chauffé à 100 °C pendant 16 heures. Après retour 23 °C, l'ensemble est dilué par 30 ml d'eau et 30 ml d'acétate d'éthyle. La phase organique est décantée, lavée par 2 fois 20 ml d'eau, séchée sur sulfate de sodium et filtrée. Le filtrat est concentré sous vide et le résidu d'évaporation est purifié sur colonne de silice (éluant : acétate d'éthyle/heptane : 1/1). On obtient une poudre jaune avec un rendement de 52%.
Point de fusion : 72-75 °C.

### 26.5) 3,4-dihydro-2,5,7,8-tétraméthyl-2-{4[(4-amminophényl)-1-pipérazinyl]-méthyl}-2H-1-benzopyran-6-ol :

Le protocole expérimental est identique celui décrit pour l'intermédiaire 1.2, l'intermédiaire 26.4 remplaçant l'intermédiaire 1.1. On obtient un solide jaune avec un rendement de 37%.
RMN ¹H (100 MHz, CDCl₃, δ) : 1,30 (s, 3H, CH₃) ; 2,00 (m, 4H, CH₂-CH₂) ; 2,10 (s, 6H, 2 x CH₃) ; 2,20 (s, 3H, CH₃) ; 2,60 (s, 2H, CH₂) ; 2,75 (m, 6H, pipérazine, NH₂) ; 3,00 (m, 4H, pipérazine) ; 4,00 (s large, 1H, OH) ; 6,70 (m, 4H, arom.).

### 26.6) N-{4-[4-[(3,4-dihydro-6-hydroxy-2,5,7,8-tétraméthyl-2H-1-benzopyran-2-yl)méthyl]-1-pipérazinyl]phényl}-2-thiophènecarboximidamide :

Le protocole expérimental est le même que celui décrit pour l'intermédiaire 1.3 excepté que le produit reste sous forme de base libre. On obtient une poudre jaune avec un rendement de 55%. Point de fusion : 100-102 °C.
RMN ¹H (400 MHz, DMSO d6, δ) : 1,25 (s, 3H, CH₃) ; 1,90 (m, 2H, Ω CH₂-CH₂) ; 2,14 (s, 3H, CH₃) ; 2,15 (s, 3H, CH₃) ; 2,20 (s, 3H, CH₃) ; 2,60 (s, 2H, NH₂) ; 2, 65 (s large, 2H, Ω. CH₂-CH₂) ; 2,80 (m, 4H, pipérazine) ; 3,15 (m, 4H, pipérazine) ; 4,80 (s large, 1H, OH) ; 6,90 (s, 4H, arom.) ; 7,05 (m, 1H, thiophène) ; 7,40 (m, 2H, thiophène).
(MH)+ : 505,3.

### Exemple 27 : chlorhydrate de 1-(2-hydroxy-4,6-diméthoxybenzoyl)-3-{4-[(imino(2-thiényl)méthyl)amino]phénoxy}azétidine :

### 27.1) 1-(diphénylméthyl)-3-(4-nitrophénoxy)azétidine :

A une suspension de 0,06 g (2,3 mmol) de NaH dans 20 ml de THF sec, sous atmosphère d'argon, on ajoute 0,5 g (2 mmol) de 1-(diphénylméthyl)-3-hydroxyazétidine. Après une heure d'agitation à 23 °C, on ajoute goutte-à-goutte au mélange réactionnel une solution de 0,29 g (2,1 mmol) de 4-fluoronitrobenzène dans 5 ml de THF sec. L'agitation est maintenue pendant 2 heures supplémentaires à 23 °C et l'ensemble est finalement versé dans 25 ml d'eau. Le produit est extrait par 2 fois 25 ml d'acétate d'éthyle, la phase organique est ensuite lavée par 2 fois 25 ml de saumure, séchée sur sulfate de magnésium, filtrée et concentrée sous vide. Le produit est purifié sur une colonne de silice (éluant : 12% d'acétate d'éthyle dans l'heptane). Les fractions pures sont évaporées pour conduire à une huile incolore avec un rendement de 40%.
RMN ¹H (CDCl₃, 400 MHz, δ) : 3,20 (m, 2H, azétidine) ; 4,50 (s, 1H, CH-(Ph)₂) ; 4,80 (m, 2H, azétidine); 4,90 (m, 1H, CH-O); 6,80 (m, 2H, H arom.); 7,20-7,50 (m, 10H, H arom.) ; 8,20 (m, 2H, H arom.).

### 27.2) 1-(diphénylméthyl)-3-(4-aminophénoxy)azétidine :

A une solution de l'intermédiaire 27.1 (1,14 g ; 3,18 mmol) dans 50 ml d'un mélange acétate d'éthyle/éthanol/acétone (2/1/2), on introduit 3,59 g (16 mmol) de SnCl₂, 2H₂O. Le mélange réactionnel est chauffé à reflux pendant 5 heures et finalement après refroidissement, concentré de moitié sous vide. Le résidu d'évaporation est ensuite versé dans 50 ml d'une solution saturée de NaHCO₃ froide et extrait par 100 ml d'acétate d'éthyle. Le mélange trouble est filtré sur célite et le filtrat est décanté. La phase organique est lavée successivement par 50 ml d'eau et 50 ml de saumure. Après séchage sur sulfate de magnésium et filtration, la solution organique est concentrée sous vide. On obtient une huile incolore avec un rendement de 75%.
RMN ¹H (CDCl₃, 400 MHz, δ) : 3,10 (m, 2H, azétidine) ; 3,40 (s large, 2H, NH₂) ; 4,40 (s, 1H, CH-(Ph)₂) ; 4,70 (m, 2H, azétidine) ; 4,75 (m, 1H, CH-O); 6,60 (s, 4H, H arom.) ; 7,10-7,40 (m, 10H, H arom.).

### 27.3) 1-(diphénylméthyl)-3-{4-[(1,1-diméthyléthoxy)carbonyl]-aminophénoxy}azétidine :

La protection de l'amine est classiquement effectuée par BocOBoc en présence de triéthylamine dans le dichlorométhane. On obtient un solide blanc avec un rendement de 77%. Point de fusion : 149-151 °C.
RMN ¹H (DMSO d6, 400 MHz, δ) : 1,40 (s, 9H, tBu) ; 2,90 (s large, 2H, azétidine) ; 3,60 (s large, 2H, azétidine) ; 4,50 (s, 1H, CH-(Ph)₂) ; 4,70 (m, 1H, CH-O); 6,70 (m, 2H, H arom.) ; 7,10-7,60 (m, 12H, H arom.) ; 9,10 (s, 1H, NH).

### 27.4) 3-{4-[(1,1-diméthyléthoxy)carbonyl]aminophénoxy}azétidine :

Le protocole expérimental utilisé est le même que celui décrit pour l'intermédiaire 1.2 sauf pour le catalyseur d'hydrogénation qui est remplacé par Pd(OH)₂. On obtient un solide blanc avec un rendement de 78%. Point de fusion : 184-186 °C.
RMN ¹H (DMSO d6, 400 MHz, δ) : 1,50 (s, 9H, tBu) ; 3,50 (m, 2H, azétidine) ; 3,70 (m, 2H, azétidine); 4,90 (m, 1H, CH-O); 6,70 (m, 2H, H arom.); 7,30 (m, 2H, H arom.) ; 9,10 (s, 1H, NH).

### 27.5) 1-(2-hydroxy-4,6-diméthoxybenzoyl)-3-{4-[(1,1-diméthyléthoxy)carbonyl]aminophénoxy}azétidine :

Le protocole expérimental utilisé est le même que celui décrit pour l'exemple 1, l'acide 2-hydroxy-4,6-diméthoxybenzoïque remplaçant l'acide 5-méthoxysalicylique. On obtient un solide blanc. Point de fusion : 95 °C.
RMN ¹H (DMSO d6, 400 MHz, δ) : 1,45 (s, 9H, Boc) ; 3,72 (s, 3H, -OCH₃) ; 3,73 (s, 3H, -OCH₃) ; 3,90 (m, 2H, CH₂, azétidine) ; 4,20 (m, 1H, CH₂, azétidine) ; 4,40 (m, 1H, CH₂, azétidine) ; 5,00 (m, 1H, CH, azétidine) ; 6,07 (d, 2H, arom., J = 8,95 Hz) ; 6,76 (d, 2H, arom., J = 8,95 Hz) ; 7,36 (m, 2H, arom.) ; 9,2 (s large, NH-Boc) ; 10,3 (s large, 1H, -OH).

### 27.6) 1-(2-hydroxy-4,6-diméthoxybenzoyl)-3-aminophénoxy-azétidine :

Dans un ballon sous atmosphère d'argon on introduit l'intermédiaire 27.5 (0,8 g ; 1,8 mmol) dans 20 ml de dichlorométhane. La solution est refroidie à l'aide d'un bain de glace et on ajoute goutte à goutte l'acide trifluoroacétique (0,7 ml ; 9,0 mmol). On laisse agiter en revenant à température ambiante durant la nuit. On évapore à sec, reprend le résidu au bicarbonate de sodium et l'extrait ensuite à l'acétate d'éthyle. La phase organique est séchée sur sulfate de sodium, filtrée et concentrée sous vide. On procède alors à une purification sur colonne de silice (éluant = chloroforme à 3% éthanol) pour conduire à un solide blanc avec un rendement de 52%. Point de fusion : 95 °C.
RMN ¹H (CDCl₃, 400 MHz, δ) ; 3,60 (m, 2H, CH₂, azétidine) ; 3,80 (s, 3H, -OCH₃) ; 3,83 (s, 3H, -OCH₃) ; 4,20 (s large, 2H, CH₂) ; 4,37 (m, 2H, CH₂, azétidine) ; 4,86 (m, 1H, CH, azétidine) ; 5,95 (s, 1H, arom.) ; 6,15 (s, 1 H, arom.) ; 6,62 (m, 4H, arom.) ; 11,61 (s large, 1H, -OH).

### 27.7) Chlorhydrate de 1-(2-hydroxy-4,6-diméthoxybenzoyl)-3-{4-[(imino(2-thiényl)méthyl)amino]phénoxy}azétidine :

Le protocole expérimental utilisé est le même que celui décrit pour l'intermédiaire 1.3, l'intermédiaire 27.6 remplaçant 2-hydroxy-5-méthoxy-N-{2-(4-aminophényl}éthyl}benzamide. On obtient un solide blanc. Point de fusion : 185-187,7 °C.
RMN ¹H (DMSO d6, 400 MHz, δ) ; 3,71 (s, 3H, -OCH₃) ; 3,73 (s, 3H, -OCH₃) ; 3,85 (m, 2H, CH₂, azétidine) ; 4,32 (m, 2H, CH₂, azétidine) ; 5,08 (m, 1H, CH, azétidine) ; 6,09 (m, 2H, arom.) ; 7,02 (m, 2H, arom.) ; 7,36 (m, 3H, arom.) ; 8,16 (m, 2H, thiophène) ; 8,74 (s large, 1H, NH⁺) ; 9,72 (s large, 1H, NH⁺) ; 10,33 (s, 1H, -OH) ; 11,39 (s large, 1H, NH⁺).
IR : ν_{C=N} (amidine) : 1590 cm⁻¹.

### Exemple 28 : chlorhydrate de N-(2-hydroxy-5-méthoxy)-4-{[2-thiényl(imino)méthyl]amino}benzène-butanamide

### 28.1) 2-amino-4-méthoxyphénol

Le protocole expérimental utilisé est le même que celui décrit pour l'intermédiaire 1.2, le 4-méthoxy-2-nitrophénol remplaçant le 2-hydroxy-5-méthoxy-N-{2-(4-nitrophényl)éthyl}-benzamide.

### 28.2) N-(2-hydroxy-5-méthoxy)-4-nitro-benzène-butanamide

Le protocole expérimental utilisé est le même que celui décrit pour l'intermédiaire 1.1, l'acide 4-(4-nitrophényl)butanoïque remplaçant l'acide 5-méthoxysalicyclique et le 2-amino-4-méthoxyphénol remplaçant le chlorohydrate de 4-nitrophénétylamine.

### 28.3) N-(2-hydroxy-5-méthoxy)-4-amino-benzène-butanamide.

Le protocole expérimental est utilisé est le même que celui décrit pour l'intermédiaire 1.2, le N-(2-hydroxy-5-méthoxy)-4 nitro-benzène-butanamide remplaçant le 2-hydroxy-5-méthoxy-N-{2-(4-nitrophényl)éthyl}-benzamide.

### 28.4) Chlorhydrate de N-(2-hydroxy-5-méthoxy)-4-{[2-thiényl(imino)méthyl] amino}benzène-butanamide.

Le protocole expérimental utilisé est le même que celui décrit pour l'exemple 1, le N-(2-hydroxy-5-méthoxy)-4-amino-benzène-butanamide remplaçant 2-hydroxy-5-méthoxy-N-{2-(4-aminophényl)éthyl}-benzamide. Point de fusion : 199,1 - 200,7 °C.
RMN ¹H (DMSO d6, 400 MHz, δ): -1,9 (m, 2H, CH₂) ; 2,45 (m, 2H, CH₂) ; 2,70 (m, 2H, CH₂) ; 3,64 (s, 3H, -OCH₃ ) ; 6,51-6,9 (m, 2H, arom.) ; 7,36 (m, 6H, arom.) ; 8,17 (m, 2H, thiophène) ; 8,88 (s large, 1H, NH⁺) ; 9,38 (s, 2H, -OH & CONH) ; 9,81 (s large, 1H, NH⁺) ; 11,52 (s large, 1H, NH⁺).
IR : ν_{C=N} (amidine) : 1662 cm⁻¹ ; ν_{C=O} (amide) : 1693 cm⁻¹.

### Exemple 48 : chlorhydrate de N-{4-[2-({2-[3,5-di(tert-butyl)-4-hydroxyphénoxy]éthyl}amino)éthyl]phényl}-2-thiophènecarboximidamide :

### 48.1) 2,6-di(tert-butyl)-4-{2-[(4-nitrophénétyl)amino]éthoxy}phénol :

Dans un ballon, sous atmosphère d'argon, on introduit 1,92 g (4,5 mmol) de 2-[3,5-di(*tert*-butyl)-4-hydroxyphénoxy]-N-(4-nitrophénétyl)acétamide (intermédiaire de la synthèse du composé 47) dans 80 ml de THF anhydre. Une solution de 13,45 ml (13,45 mmol) de BH₃ dans le THF est ajouté au mélange réactionnel et l'ensemble est agité en chauffant à reflux pendant 4 h 30. A la fin de la réaction, on ajoute 10 ml de MeOH et le chauffage est maintenu encore 30 min. Le mélange réactionnel est ensuite concentré sous vide, le résidu est repris par 30 ml d'un mélange MeOH/HCl 3N (2/1) et l'ensemble est porté à reflux une heure supplémentaire. Après retour à 22 °C, on dilue par 50 ml de CH₂Cl₂ et on additionne de la soude aqueuse 2 M jusqu'à pH basique. Après décantation, la phase organique est lavée successivement par 20 ml d'eau et 20 ml de saumure, séchée sur sulfate de sodium, filtrée et concentrée sous vide. Le produit attendu est purifié par chromatographie sur une colonne de silice (éluant : CH₂Cl₂/EtOH : 25/1). Solide jaune pâle.

### 48.2) 2-[3,5-di(tert-butyl)-4-hydroxyphénoxy]éthyl(4-nitrophénéthyl)carbamate de tert-butyle :

On dissout 1,27 g (3 mmol) de l'intermédiaire 48.1 dans un mélange de 15 ml de CH₂Cl₂ et 0,53 ml (3 mmol) de N,N-diisopropyléthylamine. L'ensemble est refroidi à l'aide d'un bain de glace avant l'addition en une portion de 0,67 g (3 mmol) de (Boc)₂O. Le mélange réactionnel est agité à 23 °C pendant 5 heures. Après concentration sous vide, le résidu est repris par 50 ml d'acétate d'éthyle et versé dans un mélange eau-glace. La phase organique est décantée, lavée successivement par 20 ml d'eau et 20 ml de saumure. Après séchage sur sulfate de sodium, filtration et concentration sous vide, on obtient un solide jaune avec un rendement quantitatif.

### 48.3) 4-aminophenéthyl{2-[3,5-di(tert-butyl)-4-hydroxyphénoxy]-éthyl}carbamate de tert-butyle :

On dissout 1,7 g (3 mmol) de l'intermédiaire 48.2 et 0,8 ml (15 mmol) d'hydrate d'hydrazine dans 50 ml d'éthanol absolu avant l'addition de 0,2 g de Nickel de Raney. Le mélange réactionnel est ensuite chauffé à reflux jusqu'à disparition totale du produit de départ (4 h 30). Après refroidissement, on ajoute une petite quantité de silice dans le ballon et le solvant est finalement éliminé sous vide. La poudre ainsi obtenue est directement déposée au sommet d'une colonne de chromatographie. Le produit est élué à l'aide d'un mélange AcOEt / Heptane : 1 / 2. Le produit attendu est obtenu sous forme d'une huile orange avec un rendement de 83%.

### 48.4) 4-{[amino(2-thiényl)méthylidène]amino}phenéthyl{2-[3,5-di(tert-butyl)-4-hydroxyphénoxy]éthyl}carbamate de tert-butyle :

Dans 50 ml d'isopropanol, on dissout 0,93 g (1,9 mmol) de l'intermédiaire 48.3 et 0,60 g (2,1 mmol) d'iodhydrate de S-méthyl-2-thiophènethiocarboximide (*Ann. Chim.* (1962) 7, 303-337). Le mélange réactionnel est agité 15 heures à 60 °C. Après évaporation du solvant sous vide, le résidu est repris par 60 ml d'AcOEt et 40 ml d'une solution saturée de Na₂CO₃. Le mélange est fortement agité et finalement décanté. La phase organique est lavée successivement par 20 ml d'eau, 20 ml de saumure, séchée sur sulfate de sodium, filtrée et concentrée sous vide. Le produit est investi directement dans l'étape suivante sans purification supplémentaire.

### 48.5) N-{4-[2-({2-[3,5-di(tert-butyl)-4-hydroxyphénoxy]-éthyl}amino)éthyl]phényl}-2-thiophènecarboximidamide :

On dissout 0,8 g (1,3 mmol) de l'intermédiaire 48.4 dans 20 ml d'éthanol et on ajoute 7 ml d'une solution 3N d'acide chlorhydrique. Le mélange est agité 1 heure à 23 °C. Après refroidissement à l'aide d'un bain de glace, la solution est rendue basique par addition de Na₂CO₃ en poudre et l'ensemble est finalement dilué par 50 ml d'AcOEt. Après agitation vigoureuse et décantation, la phase organique est lavée par 20 ml de saumure, séchée sur sulfate de sodium, filtrée et concentrée à sec. Le résidu est purifié sur une colonne de silice (éluant : AcOE(/Heptane/NH4OH : 12,5/12,5/0,5).

### 48.6) chlorhydrate de N-{4-[2-({2-[3,5-di(tert-butyl)-4-hydroxyphénoxy]-éthyl}amino)éthyl]phényl}-2-thiophènecarboximidamide :

L'intermédiaire 48.5 (0,29 g, 0,6 mmol) est dissous dans 30 ml d'éthanol absolu, l'ensemble est refroidi à l'aide d'un bain de glace avant l'addition de 2,4 ml (2,4 mmol) d'une solution 1N d'HCl dans l'éther anhydre. Après une demi-heure d'agitation à 23 °C, le solvant est évaporé sous vide pour conduire un solide rosé. Point de fusion : 151-153 °C.

### Exemple 49 : 3-{[amino(2-thiényl)méthylidène]amino}benzyl{3-[4-(diméthylamino)anilino]-3-oxopropyl}carbamate de tert-butyle :

### 49.1) 3-[4-(diméthylamino)anilino]-3-oxopropyl carbamate de tert-butyle :

Le protocole expérimental utilisé est le même que celui décrit pour l'intermédiaire 1.1, à partir de la N-Boc-β-alanine et de la N,N-diméthyl-p-phénylènediamine. Solide blanc. Point de fusion : 166-168 °C.

### 49.2) 3-amino-N-[4-(diméthylamino)phényl]propanamide :

A une solution de 3,46 g (11,3 mmol) de l'intermédiaire 49.1 dans 85 ml d'AcOEt, on ajoute 35 ml d'une solution 6N d'HCl. Le mélange réactionnel est agité 15 minutes à 23 °C. Après décantation, la phase aqueuse est recueillie, rendue basique par addition, à 0 °C, de NaOH 2N. Le produit est ensuite extrait à l'aide de 2 fois 100 ml de CH₂Cl₂. La solution organique est ensuite lavée par 25 ml de saumure, séchée sur sulfate de sodium, filtrée et concentrée sous vide. On obtient un solide beige (73%) qui est utilisé tel quel dans l'étape suivante.

### 49.3) N-[4-(diméthylamino)phényl]-3-[(3-nitrobenzyl)amino]propanamide

A un ballon contenant 100 ml de MeOH anhydre, sous atmosphère inerte, on ajoute successivement 1,15 g (5,5 mmol) de l'intermédiaire 49.2, 0,92 g (6 mmol) de 3-nitrobenzaldéhyde et 3 g de tamis moléculaire 4 Å pulvérulent préalablement activé. Le mélange réactionnel est agité vigoureusement pendant 15 heures avant l'addition, par portions, de 0,24 g (6 mmol) de NaBH₄. L'agitation est maintenue 4 heures supplémentaires avant addition de 10 ml d'eau. Après un quart d'heure, le tamis est filtré et le mélange réactionnel est extrait par 2 fois 100 ml de CH₂Cl₂. La phase organique est lavée successivement par 50 ml d'eau, 50 ml de saumure, séchée sur sulfate de sodium, filtrée et concentrée sous vide. Le résidu est purifié sur une colonne de silice (éluant : CH₂Cl₂/EtOH : 20/1). On obtient un solide beige avec un rendement de 86%.

### 49.4) 3-{[amino(2-thiényl)méthylidène]amino}benzyl{3-[4-(diméthylamino)anilino]-3-oxopropyl}carbamate de tert-butyle :

Le protocole expérimental utilisé est identique à celui décrit pour la synthèse des intermédiaires 48.2 à 48.4. On obtient un solide blanc. Point de fusion : 70-72 °C.

### Exemple 54* : chlorhydrate de 3-[(3-{[amino(2-thiényl)méthylidène]amino}benzyl)amino]-N-(1-méthyl-2,3-dihydro-1H-indol-5-yl)propanamide :

### 54.1) 1-méthyl-5-nitroindoline :

Dans un tricol de 150 ml, sous atmosphère inerte, on introduit 25 ml de DMF anhydre suivi de 0,84 g (21 mmol) de NaH à 60%. Le mélange réactionnel est refroidi à l'aide d'un bain de glace avant l'addition goutte-à-goutte d'une solution de 3,28 g (20 mmol) de 5-nitroindoline dans 5 ml de DMF anhydre. A la fin de l'addition, l'agitation est maintenue 1 heure à 23 °C, avant d'introduire goutte-à-goutte une solution de 1,31 ml (21 mmol) de MeI dans 5 ml de DMF anhydre. L'agitation est poursuivie pendant 15 heures à 23 °C. La réaction est finalement neutralisée, à 0 °C, par 20 ml d'une solution saturée de NH₄Cl. Le mélange réactionnel est ensuite dilué par 20 ml d'eau et 50 ml d'AcOEt. Après décantation, la phase organique est lavée successivement par 20 ml d'eau, 20 ml de saumure, séchée sur sulfate de magnésium, filtrée et concentrée sous vide. On obtient une poudre jaune foncée.

### 54.2) 1-méthyl-5-aminoindoline :

A un mélange de 2,84 g (15,9 mmol) de 1-méthyl-5-nitroindoline et de 4 ml (80 mmol) d'hydrate d'hydrazine dans 60 ml d'éthanol absolu, on ajoute environ 400 mg de nickel de Raney. Le mélange réactionnel est chauffé à reflux pendant 5 heures. Après retour à 23 °C, on ajoute un peu de silice dans le ballon et on évapore le solvant sous vide. Le résidu d'évaporation est placé directement au sommet d'une colonne de chromatographie. Le produit attendu est élué à l'aide d'un mélange Heptane/AcOEt (3/7). On obtient une poudre violette (65%) qui est investie directement dans l'étape suivante.

### 54.3) chlorhydrate de 3-[(3-{[amino(2-thiényl)méthylidène]amino}-benzyl)amino]-N-(1-méthyl-2,3-dihydro-1H-indol-5-yl)propanamide :

Le protocole expérimental utilisé est le même que celui décrit pour le composé 49, la 1-méthyl-5-aminoindoline remplaçant la N,N-diméthyl-p-phénylènediamine. Déprotection et salification sont effectuées selon le protocole expérimental décrit pour l'intermédiaire 48.5. On obtient un solide beige clair. Point de fusion : 134-136 °C.

### Exemple 55* : chlorhydrate de 3-[(3-{[amino(2-thiényl)méthylidène]amino}benzyl)amino]-N-(1-benzyl-2,3-dihydro-1H-indol-5-yl)propanamide :

Le protocole expérimental utilisé est le même que celui décrit pour le composé 54, le bromure de benzyle remplaçant l'iodométhane. On obtient un solide blanc. Point de fusion : 193-195 °C.

### Exemple 56* : 3-[(3-{[amino(2-thiényl)méthylidène]amino}benzyl)amino}-N-[1-(1-naphthylméthyl)-2,3-dihydro-1H indol-5-yl]propanamide :

Le protocole expérimental utilisé est le même que celui décrit pour le composé 54, le 1-(chlorométhyl)-naphtalène remplaçant l'iodométhane. On obtient un solide beige. Point de fusion: 185-187°C.

### Exemple 68 : N'-[4-(2-{[(2-hydroxy-4,6-diméthoxyphényl)méthylidène]amino}éthyl)phényl]-2-thiophènecarboximidamide :

La formation de l'imine entre le 2-hydroxy-4,6-diméthoxybenzaldéhyde et la 4-nitrophénétylamine est effectuée selon le protocole expérimental utilisé pour la synthèse de l'intermédiaire 49.3, à l'exception de l'addition de NaBH₄. Les autres étapes sont identiques à celles décrites pour les synthèses des intermédiaires 27.2 et 1.3 (le produit restant sous forme de base libre). Huile de couleur jaune vif qui cristallise partiellement.

### Exemple 70 : N'-{4-[4-phényl-3,6-dihydro-1(2H)-pyridinyl]phényl}-2-thiophènecarboximidainide :

### 70.1) 1-(4-nitrophényl)-4-phényl-1,2,3,6-tétrahydropyridine :

On chauffe à 100 °C, pendant 24 heures, un mélange de 0,5 g (2,5 mmol) du chlorhydrate de 4-phényl-1,2,3,6-tétrahydropyridine, 0,51 ml (2,8 mmol) de diisopropyléthylamine, 0,71 g (5 mmol) de K₂CO₃, 0,40 g ((2,8 mmol) de 4-fluoronitrobenzène dans 10 ml de DMF. A la fin de la réaction, le mélange est versé sur 25 ml d'une solution saturée de NaHCO₃ et le produit est extrait à l'aide de 25 ml d'AcOEt. Après décantation, la solution organique est lavée par 2 fois 20 ml d'eau suivi de 20 ml de saumure. Séchage sur sulfate de magnésium, filtration et évaporation du solvant sous vide conduisent à un solide jaune avec un rendement de 42%. Point de fusion : 185-192 °C (décomposition).

### 70.2) N'-{4-[4-phényl-3,6-dihydro-1(2H)-pyridinyl]phényl}-2-thiophènecarboximidamide :

Le protocole expérimental utilisé est le même que celui décrit pour les intermédiaires 27.2 et 1.3 (le produit restant sous forme de base libre).

### Exemple 71 : dichlorhydrate de N'-(4-{2-[4-phényl-3,6-dihydro-1(2H)-pyridinyl]éthyl}phényl)-2-thiophénecarboximidamide :

### 71.1)1 1-(4-nitrophenéthyl)-4-phényl-1,2,3,6-tétrahydropyridine :

A une solution de 1,0 g (5,98 mmol) de 4-nitrophenéthyl alcool dans 25 ml de CH₂Cl₂, on ajoute 1,29 g (6,6 mmol) du chlorhydrate de 4-phényl-1,2,3,6-tétrahydropyridine, 0,92 ml (6,6 mmol) de Et₃N et 1,62 ml (6,6 mmol) de tributylphosphine. Le mélange réactionnel est agité vigoureusement à 23 °C et on ajoute goutte-à-goutte 1,04 ml (6,6 mmol) de diéthylazodicarboxylate. Après 4 heures, la réaction est stoppée par addition de 5 ml d'une solution saturée de NH₄Cl. Après décantation, la phase aqueuse est extraite par 2 fois 5 ml de CH₂Cl₂, les phases organiques sont combinées et lavées par 2 fois 5 ml d'eau, 2 fois 5 ml de saumure, séchées sur sulfate de magnésium, filtrées et concentrées sous vide. Le résidu est purifié sur colonne de silice (éluant : Heptane/AcOEt : 9/1 à 7/3) pour conduire à 1,20 g du produit attendu.

### 71.2) dichlorhydrate de N'-(4-{2-[4-phényl-3,6-dihydro-1(2H)-pyridinyl]éthyl}phényl)-2-thiophènecarboximidamide :

Le protocole expérimental utilisé est le même que celui décrit pour les intermédiaires 27.2 et 1.3. Solide jaune pale. Point de fusion : 185 °C (décomp.)

### Exemple 72 : N'-{4-[(1-benzhydryl-3-azetidinyl)oxy]phényl}-2-thiophènecarboximidamide :

Le protocole expérimental utilisé est le même que celui décrit pour l'intermédiaire 1.3 (le produit étant isolé sous forme de base libre), l'intermédiaire 27.2 remplaçant l'intermédiaire 1.2. Solide blanc. Point de fusion : 210-211 °C.

### Exemple 73 : iodhydrate de N'-[4-(2-quinolinylméthoxy)phényl]-2-thiophènecarboximidamide:

### 73.1) 2-[(4-nitrophénoxy)méthyl]quinoline :

Sous atmosphère d'argon, on introduit dans un ballon contenant 25 ml de DMF anhydre 0,4 g (11 mmol) de NaH à 60%. Une solution de 1,38 g (10 mmol) de 4-nitrophénol dans 5 ml de DMF anhydre est ajoutée goutte-à-goutte, sous agitation à 23°C. Après une demi-heure une solution de 1,77 g (10 mmol) de 2-(chlorométhyl)-quinoline dans 5 ml de DMF anhydre est ajoutée goutte-à-goutte et l'agitation est poursuivie pendant 15 heures en chauffant le mélange à 90°C. A la fin de la réaction, l'ensemble est versé sur de la glace et un précipité abondant se forme. La suspension est filtrée, rincée par 2 fois 25 ml d'eau suivi de 20 ml d'éther éthylique. On obtient ainsi un solide blanc avec un rendement de 75%. Point de fusion : 154-155 °C.

### 73.2) iodhydrate de N'-[4-(2-quinolinylméthoxy)phényl]-2-thiophènecarboximidamide :

Le protocole expérimental utilisé est le même que celui décrit pour les intermédiaires 1.2 et 1.3, excepté que l'iodhydrate précipité spontanément dans la réaction, il est donc isolé pur lors d'une simple filtration.

### Exemple 77 : iodhydrate de N'-{4-[2-({[4-(diméthylamino)anilino]carbonyl}amino)éthyl]phényl}-2-thiophènecarboximidamide :

### 77.1) N-[4-(diméthylamino)phényl]-N'-(4-nitrophenéthyl)urée :

Dans un tricol de 100 ml, sous atmosphère d'argon, on dissout 0,9 g (3,3 mmol) de triphosgène dans 5 ml de CH₂Cl₂ anhydre, la solution est agitée à 23 °C. A l'aide d'une seringue motorisée, on ajoute goutte-à-goutte en 1 heure, une solution de 2,09 g (10 mmol) de dichlorhydrate de N,N-diméthyl-p-phénylènediamine et de 5,2 ml (30 mmol) de diisopropyléthylamine dans 20 ml de CH₂Cl₂ anhydre. Dix minutes après la fin de l'addition, on ajoute en une portion, une solution de 1,36 g (10 mmol) de 2-(4-aminophényl)éthylamine et de 1,8 ml de diisopropyléthylamine dans 5 ml de CH₂Cl₂ anhydre. Après 2 heures d'agitation à 23 °C, le précipité formé est éliminé par filtration, le filtrat est lavé par 10 ml d'eau suivi de 10 ml de saumure. La solution organique est séchée sur sulfate de sodium, filtrée et concentrée sous vide. Le résidu obtenu est purifié par passage rapide sur une colonne de silice (éluant : AcOEt). Solide blanc.

### 77.2) iodhydrate de N'-{4-[2-({[4-(diméthylamino)anilino]carbonyl}amino)éthyl]phényl}-2-thiophènecarboximidamide :

Le protocole expérimental utilisé est le même que celui décrit pour la synthèse des intermédiaires 1.2 et 1.3 excepté le fait que l'iodhydrate, précipitant spontanément dans la réaction, est isolé pur lors d'une simple filtration. Solide jaune. MH+ = 408,2.

### Exemple 78 : chlorhydrate de N-{[1-(4-{[amino(2-thiényl)méthylidène]amino}phényl)-cyclobutyl]méthyl}-6-hydroxy-2,5,7,8-térraméthyl-2-chromanecarboxamide :

### 78.1) 2-[4-(2,5-diméthyl-1H-pyrrol-1-yl)phényl]acétonitrile :

Dans un ballon de 500 ml, on dissout 20 g (0,15 mol) de cyanure de p-aminobenzyle, 19 ml (0,17 mol) de 2,5-dicétohexane et 2,88 g (0,015 mol) d'acide p-toluène sulfonique hydraté. Ce mélange est chauffé à reflux, l'eau formée est piégée à l'aide d'un appareil de Dean Stark. A la fin de la réaction, le solvant est évaporé sous vide et le résidu est repris par 300 ml d'AcOEt. La solution organique est lavée successivement par 2 fois 200 ml d'eau, 200 ml d'une solution saturée de Na₂CO₃, 200 ml d'eau, 200 ml d'une solution saturée de KHSO₄ et finalement 200 ml d'eau et 200 ml de saumure. Après séchage sur sulfate de sodium, filtration et concentration sous vide, le résidu est purifié rapidement sur une colonne de silice. On obtient un solide beige clair avec un rendement de 34%. Point de fusion: 112-113 °C.

### 78.2) 1-[4-(2,5-diméthyl-1H pyrrol-1-yl)phényl]cyclopentanecarbonitrile :

Dans un tricol de 100 ml, sous atmosphère d'argon, contenant 20 ml de DMSO anhydre, on introduit 0,88 g (22 mmol) de NaH à 60%. A cette suspension, sous agitation, on ajoute aussi rapidement que possible une solution de 2,1 g (10 mmol) de l'intermédiaire 78.2 et de 2,23 ml (11 mmol) de 1,3-dibromopropane dans 10 ml de DMSO anhydre. La vitesse d'addition est réglée en fonction de la température du milieu réactionnel qui doit rester vers 25-35 °C. A la fin de la réaction (1 h 30), le contenu du ballon est versé dans 100 ml d'un mélange eau + glace. Le produit est extrait par 50 ml d'AcOEt et la solution organique est lavée successivement par 5 fois 20 ml d'eau, 20 ml de saumure, séchée sur sulfate de sodium, filtrée et concentrée sous vide. Le résidu est purifié sur une colonne de silice (éluant : Heptane/AcOEt : 9/1). On obtient un solide blanc avec un rendement de 55%. Point de fusion : 117-120 °C.

### 78.3) {1-[4-(2,5-diméthyl-1H-pyrrol-1-yl)phényl]cyclopentyl}méthanamine :

Dans un tricol de 100 ml, sous atmosphère d'argon, contenant 20 ml (20 mmol) d'une solution 1M de LiAlH₄ dans le THF anhydre, on introduit goutte-à-goutte, à 0 °C, une solution de 2,03 g (8,1 mmol) de l'intermédiaire 78.2 dans 20 ml de THF anhydre. A la fin de l'addition, le mélange réactionnel est agité 3 heures à 23 °C. Le mélange réactionnel est ensuite refroidi à l'aide d'un bain de glace et l'excès de LiAlH₄ est détruit en additionnant lentement 20 ml d'AcOEt suivi de 10 ml d'une solution aqueuse 1M de NaOH. Après une demi-heure d'agitation vigoureuse à 23 °C, le contenu du tricol est filtré à travers de la célite. Le filtrat est dilué par 40 ml d'AcOEt et 20 ml d'eau. Après décantation, la solution organique est lavée par 20 ml d'une solution de NaOH 1M, 20 ml de saumure, séchée sur sulfate de sodium, filtrée et concentrée sous vide. Le résidu est purifié sur une colonne de silice (éluant : CH₂Cl₂/EtOH : 9/1 à 8/2). On obtient une huile jaune avec un rendement de 64%.

### 78.4) N-({1-[4-(2,5-diméthyl-1H-pyrrol-1-yl)phényl]cyclopentyl}méthyl)-6-hydroxy-2,5,7,8-tetraméthyl-2-chromanecarboxamide :

Le protocole expérimental utilisé est le même que celui décrit pour l'intermédiaire 26.1, l'intermédiaire 78.3 remplaçant la 1-benzyl pipérazine. On obtient un solide blanc avec un rendement de 79%. Point de fusion : 90-94 °C.

### 78.5) N-{[1-(4-aminophényl)cyclopentyl]méthyl}-6-hydroxy-2,5,7,8-tetraméthyl-2-chromanecarboxamide :

On chauffe à 100 °C, pendant 72 heures, une solution de 0,49 g (1 mmol) de l'intermédiaire 78.4 et de 1,05 g (15 mmol) de chlorhydrate d'hydroxylamine dans un mélange de 13 ml d'isopropanol et de 1 ml d'eau. Après refroidissement, le solvant est évaporé sous vide et le résidu est repris par 20 ml d'AcOEt et 20 ml d'eau. La phase organique est décantée et lavée par 20 ml d'eau suivi de 20 ml de saumure, séchée sur sulfate de sodium, filtrée et concentrée sous vide. Le résidu d'évaporation est purifié sur colonne de silice (éluant : Heptane/AcOEt : 6/4). Le produit attendu est obtenu sous forme d'une huile jaune avec un rendement de 50%.

### 78.6) chlorhydrate de N-{[1-(4-{[amino(2-thiényl)méthylidène]amino}phényl)cyclobutyl]méthyl}-6-hydroxy-2,5,7,8-tetraméthyl-2-chromanecarboxamide :

Le protocole expérimental utilisé est le même que celui décrit pour l'intermédiaire 1.3, l'intermédiaire 78.5 remplaçant l'intermédiaire 1.2. Solide beige. Point de fusion: 185-187 °C.

### Exemple 79 : chlorhydrate de N'-{4-[4-(5-méthoxy-1H-indol-3-yl)-1-pipéridinyl]phényl}-2-thiophènecarboximidamide

### 79.1) 5-méthoxy-3-(1,2,3,6-tétrahydro-4-pyridinyl)-1H-indole :

Le protocole expérimental utilisé est décrit dans la littérature (*Eur. J. Med. Chem.* (1987) **22**, 33-43) à partir du 5-méthoxy-1*H*-indole et de la 4,4-pipéridinediol.

### 79.2) 5-méthoxy-3-[1-(4-nitrophényl)-1,2,3,6-tétrahydro-4-pyridinyl]-1H-indole :

Le protocole expérimental utilisé est le même que celui décrit pour l'intermédiaire 26.4 en partant de l'intermédiaire 79.1.

### 79.3) 4-[4-(5-méthoxy-1H-indol-3-yl)-1-pipéridinyl]phénylamine :

On additionne, par petites portions, 9,2 g de Zinc à une solution, refroidie à l'aide d'un bain de glace, de 0,7 g (2 mmol) de l'intermédiaire 79.2 dans 35 ml d'acide acétique. Ce mélange est agité 18 heures à 20 °C avant d'être filtré. Le filtrat est concentré sous vide et les traces d'acide acétique sont éliminées par coévaporation en présence de toluène. Le résidu d'évaporation est finalement dissous dans 100 ml de CH₂Cl₂. La solution organique obtenue est ensuite lavée successivement par 50 ml d'eau et 50 ml de saumure. Après séchage sur MgSO₄, filtration et évaporation du solvant sous vide, le résidu est utilisé tel quel dans l'étape suivante.

### 79.4) chlorhydrate de N'-{4-[4-(5-méthoxy-1H-indol-3-yl)-1-pipéridinyl]-phényl}-2-thiophènecarboxunidamide :

Le protocole expérimental utilisé est le même que celui décrit pour l'intermédiaire 1.3, l'intermédiaire 79.3 remplaçant l'intermédiaire 1.2. Solide orange. Point de fusion : 209-213 °C.

### Exemple 81* : 4-(4-{[amino(2-thiényl}méthylidène]amino}phényl)-N-{1-[3-(dimethylamino)propyl]-2,3-dihydro-1H-indol-5-yl}butanamide

### 81.1) N,N-diméthyl-3-(5-nitro-2,3-dihydro-1H-indol-1-yl)-1-propanamine:

Dans un bicol de 250 ml, sous atmosphère d'argon, contenant une suspension de 2,4 g (60 mmol) de NaH (60%) dans 60 ml de DMF, on introduit par portions 4,92 g (30 mmol) de 5-nitroindoline. Le dégagement vigoureux d'hydrogène s'accompagne de la formation d'une suspension rouge. L'agitation est maintenue 30 minutes à 23 °C avant l'addition, par portions, de 4,74 g (30 mmoles) du chlorhydrate du chlorure de 3-diméthylaminopropyle. Le mélange réactionnel est agité et chauffé à 60 °C pendant 18 heures. L'ensemble est finalement versé sur 200 ml d'un mélange eau + glace. Le produit est ensuite extrait par 2 fois 100 ml d'AcOEt. La solution organique est lavée successivement par 100 ml d'eau et 100 ml de saumure. Après séchage sur MgSO₄ et concentration sous vide, le résidu d'évaporation est purifié par chromatographie éclair sur colonne de silice (éluant : AcOEt/MeOH : 100/0 à 0/100). On obtient une huile jaune foncée avec un rendement de 48%.

### 81.2) 1-[3-(diméthylamino)propyl]-5-indolinamine :

Le protocole expérimental utilisé est le même que celui décrit pour l'intermédiaire 54.2, l'intermédiaire 81.1 remplaçant l'intermédiaire 54.1. Huile violette.

### 81.3) 4-(4-{[amino(2-thiényl)méthylidène]amino}phényl)-N-{1-[3-(diméthylamino)propyl]-2,3-dihydro-1H-indol-5-yl}butanamide :

Le protocole expérimental utilisé est le même que celui décrit pour l'Exemple 1, à partir de l'intermédiaire 81.2 et de l'acide 4-nitrophénylbutanoïque.

### Exemple 82* : 3-[(5-{[amino(2-thiényl)méthylidène]amino}-2-méthoxybenzyl)amino}-N-[1-(1-naphthylméthyl)-2,3-dihydro-1H-indol-5-yl]propanamide :

Le protocole expérimental utilisé est le même que celui décrit pour l'Exemple 56, le 2-méthoxy-5-nitrobenzaldehyde (*J. Org. Chem.* (1993) **58**, 1385-1392) remplaçant le 3-nitrobenzaldéhyde.

### Etude pharmacologique des produits de l'invention

### Etude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl. Acad Sci. USA,* (1990) **87**: 682-685). Des cervelets de rats Sprague-Dawley (300g - Charles River) sont rapidement prélevés, disséqués à 4 °C et homogénéisés dans un volume de tampon d'extraction (HEPES 50 mM, EDTA 1 mM, pH 7,4, pepstatin A 10 mg/ml, leupeptine 10 mg/ml). Les homogénats sont ensuite centrifugés à 21000 g pendant 15 min à 4 °C. Le dosage se fait dans des tubes à essai en verre dans lesquels sont distribués 100 µl de tampon d'incubation contenant 100 mM d'HEPES (pH 7,4), 2 mM d'EDTA, 2.5 mM de CaCl₂, 2 mM de dithiotréitol, 2 mM de NADPH réduit et 10 µg/ml de calmoduline. On ajoute 25 µl d'une solution contenant 100 nM de [³H]L-arginine (Activité spécifique : 56.4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide. Les composés des exemples 1 à 7, 10 à 12, 19, 21, 22, 26 à 28, 31, 32, 38 à 42, 48, 50 à 62, 65, 66, 68, 71 et 73 décrits ci-dessus présentent une CI₅₀ inférieure à 3,5 µM.

### Etude des effets sur la péroxidation lipidique du cortex cérébral de rat

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol.* (1990) **186** : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g/ 15 ml et centrifugé à 515 g pendant 10 minutes à 4 °C. Le surnageant est utilisé immédiatement pour la détermination de la péroxidation lipidique. L'homogénat de cortex cérébral de rat (500 µl) est incubé à 37 °C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. *European J. Pharmacol.* (1995) **285**, 203-206). Les composés des exemples 1, 3, 14 à 16, 25, 29, 32 à 34, 37 à 39, 41, 42, 44 à 48, 50, 51, 54 à 57, 61, 62, 70, 71, 74 et 76 à 78 décrits ci-dessus présentent une CI₅₀ inférieure à 30 µM.

## Revendications

1. Produit de formule générale **(I')** 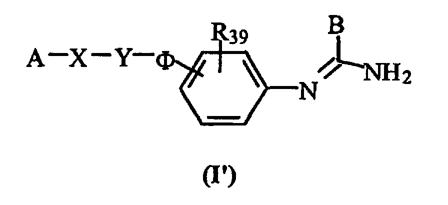 dans laquelle
A représente un radical 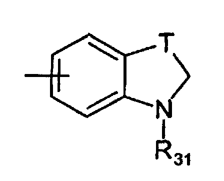 dans lequel T représente un radical -(CH₂)ₖ-, k représentant 1 ou 2, et R₃₁ représente un radical alkyle linéaire ou ramifié ayant de 1 à 6 atomes de carbone, ou l'un des radicaux aminoalkyle, alkylaminoalkyle, dialkylaminoalkyle, arylalkyle, diarylalkyle ou *bis*-arylalkyle dont le radical alkyle est linéaire ou ramifié et compte de 1 à 6 atomes de carbone ;
B représente un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
ou encore B représente un radical NR₃₄R₃₅, dans lequel R₃₄ et R₃₅ représentant, indépendamment, un atome d'hydrogène ou un radical alkyle linéaire ou ramifié ayant de 1 à 6 atomes de carbone, ou R₃₄ et R₃₅ forment avec l'atome d'azote un hétérocycle non aromatique de cinq à six chaînons, les éléments de la chaîne étant choisis parmi un groupe composé de -CH₂-, -NH-, -O- ou -S- ;
X représente une liaison ou un radical -(CH₂)ₘ-, -(CH₂)ₘ-CO, -O-(CH₂)ₘ-, -S-(CH₂)ₘ-, -NR₃₆-(CH₂)ₘ-, -CO-NR₃₆-, -O-(CH₂)ₘ-CO-, -S-(CH₂)ₘ-CO-, -NR₃₆-(CH₂)ₘ-CO-, -(CH₂)ₘ-C(OH)(CH₃)-CO-, -CH=CH- ou -CH=N-;
Y représente une liaison ou un radical -(CH₂)ₙ- ou -(CH₂)ᵣ-Q-(CH₂)ₛ-,
Q représentant un radical pipérazine, homopipérazine, 2-méthylpipérazine, 2,5-diméthylpipérazine, pipéridine, 1,2,3,6-tétrahydropyridine, pyrrolidine, azétidine, thiazolidine ou un cycle carboné saturé ayant de 3 à 7 chaînons ;
Φ représente une liaison ou un radical -(CH₂)ₚ-O-(CH₂)_{q}-, -(CH₂)ₚ-S-(CH₂)_{q}-, -(CH₂)ₚ-NR₃₇-(CH₂)_{q}-, -(CH₂)ₚ-CO-NR₃₇-(CH₂)_{q}- OU -CO-(CH₂)ₚ-NR₃₇-(CH₂)_{q}-;
R₃₆ et R₃₇ représentent, indépendamment, un atome d'hydrogène, un radical alkyle linéaire ou ramifié ayant de 1 à 6 atomes de carbone ou un radical -CO-R₃₈ dans lequel R₃₈ représente un radical alkyle ou alkoxy linéaire ou ramifié ayant de 1 à 6 atomes de carbone ;
R₃₉ représente un atome d'hydrogène ou un radical alkyle ou alkoxy linéaire ou ramifié ayant de 1 à 6 atomes de carbone ;
m, n, p, q, r et s étant des entiers de 0 à 6 ;
ou encore les sels des produits de formule générale **(I')**.

2. Produit selon la revendication 1, **caractérisé en ce que** R₃₁ représente le radical méthyle, benzyle ou naphtylméthyle.

3. Produit selon la revendication 1, **caractérisé en ce qu'**il est choisi parmi les composés suivants :
- 3-[(3-{[amino(2-thiényl)méthylidène]amino}-benzyl)amino]-N-(1-méthyl-2,3-dihydro-1H-indol-5-yl)propanamide ;
- 3-[(3-{[amino(2-thiényl)méthylidène]amino}-benzyl)amino]-N-(1-benzyl-2,3-dihydro-1H-indol-5-yl)propanamide ;
- 3-[(3-{[amino(2-thiényl)méthylidène]amino}benzyl)amino]-N-[1-(1-naphthylméthyl)-2,3-dihydro-1H-indol-5-yl]propanamide ;
- 4-(4-{[amino(2-thiényl)méthylidène]amino}phényl)-N-{1-[3-(diméthylamino)propyl]-2,3-dihydro-1H-indol-5-yl}butanamide ;
- 3-[(5-{[amino(2-thiényl)méthylidène]amino}-2-méthoxybenzyl)amino]-N-[1-(1-naphthylméthyl)-2,3-dihydro-1H-indol-5-yl]propanamide ;
et les sels de ces derniers.

4. A titre de produits industriels nouveaux, les composés de formule générale **(IS')** 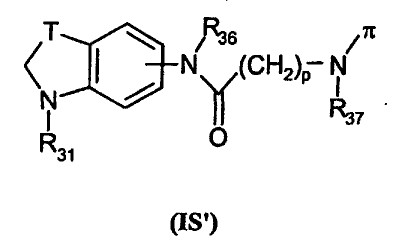 dans laquelle
π représente un atome d'hydrogène ou un groupe protecteur de type carbamate ;
R₃₆ et R₃₇ représentent, indépendamment, un atome d'hydrogène, un radical alkyle linéaire ou ramifié ayant de 1 à 6 atomes de carbone ou un radical -CO-R₃₈ dans lequel R₃₈ représente un radical alkyle ou alkoxy linéaire ou ramifié ayant de 1 à 6 atomes de carbone ;
T représente un radical -(CH₂)ₖ-, k représentant 1 ou 2 ;
R₃₁ représente un radical alkyle linéaire ou ramifié ayant de 1 à 6 atomes de carbone, ou arylalkyle, diarylalkyle, bis-arylalkyle, aminoalkyle, alkylaminoalkyle, ou dialkylaminoalkyle, dont les radicaux alkyles sont linéaires ou ramifiés et comptent de 1 à 6 atomes de carbone ;
et p est un entier de 0 à 6.

5. A titre de médicament, un produit de formule générale **(I')** selon la revendication 1, ou un sel pharmaceutiquement acceptable dudit produit.

6. Composition pharmaceutique contenant à titre de principe actif au moins un produit selon la revendication 1, ou un sel pharmaceutiquement acceptable dudit produit.

7. Utilisation d'un produit de formule générale **(I')** selon la revendication 1, ou d'un sel pharmaceutiquement acceptable dudit produit, pour préparer un médicament ayant à la fois une activité d'inhibition de la NO synthase et d'inhibition de la peroxydation lipidique.

8. Utilisation d'un produit de formule générale **(I')** selon la revendication 1, ou d'un sel pharmaceutiquement acceptable dudit produit, pour préparer un médicament destiné à traiter les maladies cardio-vasculaires et cérébro-vasculaires.

9. Utilisation d'un produit de formule générale **(I')** selon la revendication 1, ou d'un sel pharmaceutiquement acceptable dudit produit, pour préparer un médicament destiné à traiter les troubles du système nerveux central ou périphérique.

10. Utilisation selon la revendication 9, **caractérisée en ce que** le médicament préparé est destiné à traiter des maladies neurodégénératives.

## Claims

1. Product of general formula **(I')** 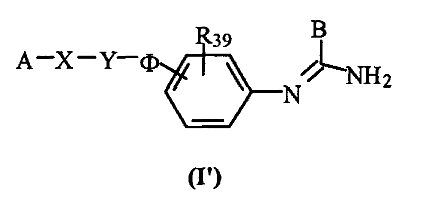 in which
A represents a 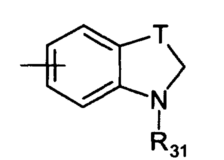 radical, in which T represents a -(CH₂)ₖ- radical, k representing 1 or 2, and R₃₁ represents a linear or branched alkyl radical with 1 to 6 carbon atoms, or one of the aminoalkyl, alkylaminoalkyl, dialkylaminoalkyl, arylalkyl, diarylalkyl or *bis*-arylalkyl radicals, the alkyl radical of which is linear or branched and contains 1 to 6 carbon atoms;
B represents a linear or branched alkyl radical with 1 to 6 carbon atoms, carbocyclic or heterocyclic aryl with 5 or 6 members containing 1 to 4 heteroatoms chosen from O, S, N and in particular the thiophene, furane, pyrrole or thiazole radicals, the aryl radical being optionally substituted by one or more groups chosen from the linear or branched alkyl, alkenyl or alkoxy radicals with 1 to 6 carbon atoms,
or also B represents an NR₃₄R₃₅ radical, in which R₃₄ and R₃₅ representing, independently, a hydrogen atom or a linear or branched alkyl radical with 1 to 6 carbon atoms, or R₃₄ and R₃₅ form with the nitrogen atom a non aromatic heterocycle with five to six members, the elements of the chain being chosen from a group composed of -CH₂-, -NH-, -O- or -S-;
X represents a bond or a -(CH₂)ₘ-, -(CH₂)ₘ-CO, -O-(CH₂)ₘ-, -S-(CH₂)ₘ-, -NR₃₆-(CH₂)ₘ-, -CO-NR₃₆-, -O-(CH₂)ₘ-CO-, -S-(CH₂)ₘ-CO-, -NR₃₆-(CH₂)ₘ-CO-, -(CH₂)ₘ-C(OH)(CH₃)-CO-, -CH=CH- or -CH=N- radical;
Y represents a bond or a -(CH₂)ₙ- or -(CH₂)ᵣ-Q-(CH₂)ₛ- radical,
Q representing a piperazine, homopiperazine, 2-methylpiperazine, 2,5-dimethylpiperazine, piperidine, 1,2,3,6-tetrahydropyridine, pyrrolidine, azetidine, thiazolidine radical or a saturated carbon-containing ring with 3 to 7 members;
Φ represents a bond or a -(CH₂)ₚ-O-(CH₂)_{q}-, -(CH₂)ₚ-S-(CH₂)_{q}-, -(CH₂)ₚ-NR₃₇-(CH₂)_{q}-, -(CH₂)ₚ-CO-NR₃₇-(CH₂)_{q}- or -CO-(CH₂)ₚ-NR₃₇-(CH₂)_{q}-radical;
R₃₆ and R₃₇ represent, independently, a hydrogen atom, a linear or branched alkyl radical with 1 to 6 carbon atoms or a -CO-R₃₈ radical in which R₃₈ represents a linear or branched alkyl or alkoxy radical with 1 to 6 carbon atoms;
R₃₉ represents a hydrogen atom or a linear or branched alkyl or alkoxy radical with 1 to 6 carbon atoms;
m, n, p, q, r and s being integers from 0 to 6;
or also the salts of the products of general formula **(I')**.

2. Product according to claim 1, **characterized in that** R₃₁ represents the methyl, benzyl or naphthylmethyl radical.

3. Product according to claim 1, **characterized in that** it is chosen from the following compounds:
- 3-[(3-{[amino(2-thienyl)methylidene]amino}-benzyl)amino]-N-(1-methyl-2,3-dihydro-1H-indol-5-yl)propanamide;
- 3-[(3-{[amino(2-thienyl)methylidene]amino}-benzyl)amino]-N-(1-benzyl-2,3-dihydro-1H-indol-5-yl)propanamide;
- 3-[(3-{[amino(2-thienyl)methylidene]amino}benzyl)amino]-N-[1-(1-naphthylmethyl)-2,3-dihydro-1H-indol-5-yl]propanamide;
- 4-(4-{[amino(2-thienyl)methylidene]amino}phenyl)-N-{1-[3-(dimethylamino)propyl]-2,3-dihydro-1H-indol-5-yl}butanamide;
- 3-[(5-{[amino(2-thienyl)methylidene]amino}-2-methoxybenzyl)amino]-N-[1-(1-naphthylmethyl)-2,3-dihydro-1H-indol-5-yl]propanamide;
and the salts of the latter.

4. As new industrial products, the compounds of general formula **(IS')** 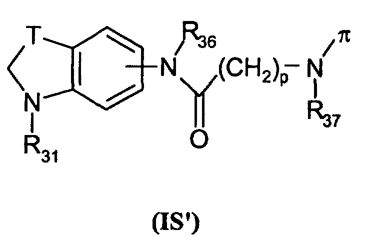 in which
π represents a hydrogen atom or a protective group of carbamate type;
R₃₆ and R₃₇ represent, independently, a hydrogen atom, a linear or branched allyl radical with 1 to 6 carbon atoms or a -CO-R₃₈ radical in which R₃₈ represents a linear or branched alkyl or alkoxy radical with 1 to 6 carbon atoms;
T represents a -(CH₂)ₖ- radical, k representing 1 or 2;
R₃₁ represents a linear or branched alkyl radical with 1 to 6 carbon atoms, or arylalkyl, diarylalkyl, bis-arylalkyl, aminoalkyl, alkylaminoalkyl or dialkylaminoalkyl, the alkyl radicals of which are linear or branched and contain 1 to 6 carbon atoms;
and p is an integer from 0 to 6.

5. As a medicament, a product of general formula **(I')** according to claim 1, or a pharmaceutically acceptable salt of said product.

6. Pharmaceutical composition containing as active ingredient at least one product according to claim 1, or a pharmaceutically acceptable salt of said product.

7. Use of a product of general formula **(I')** according to claim 1, or of a pharmaceutically acceptable salt of said product, for preparing a medicament having both an inhibition activity on NO synthase and an inhibition activity on lipidic peroxidation.

8. Use of a product of general formula (I') according to claim 1, or of a pharmaceutically acceptable salt of said product, for preparing a medicament intended to treat cardiovascular and cerebrovascular diseases.

9. Use of a product of general formula **(I')** according to claim 1, or of a pharmaceutically acceptable salt of said product, for preparing a medicament intended to treat disorders of the central or peripheral nervous system.

10. Use according to claim 9, **characterized in that** the medicament prepared is intended to treat neurodegenerative diseases.

## Patentansprüche

1. Produkt der allgemeinen Formel (I') 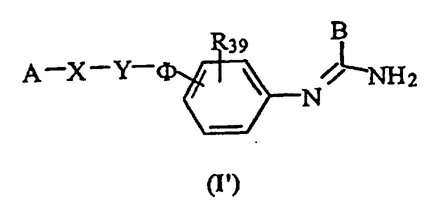 in der
A einen Rest 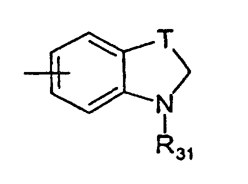 darstellt, in dem T einen Rest -(CH₂)ₖ- darstellt, wobei k 1 oder 2 darstellt,. R₃₁ einen verzweigten oder unverzweigten Alkylrest mit 1 bis 6 Kohlenstoffatomen oder einen der Reste Aminoalkyl, Alkylaminoalkyl, Dialkylaminoalkyl, Arylalkyl, Diarylalkyl oder *bis*-Arylalkyl darstellt, deren Alkylrest verzweigt oder unverzweigt ist und 1 bis 6 Kohlenstoffatome zählt;
B einen verzweigten oder unverzweigten Alkylrest mit 1 bis 6 Kohlenstoffatomen, einen carbocyclischen oder heterocyclischen Arylrest mit 5 oder 6 Kettengliedern, die 1 bis 4 Heteroatome enthalten, die aus O, S, N ausgewählt sind, und insbesondere die Reste Thiophen, Furan, Pyrrol oder Thiazol darstellt, wobei der Arylrest ggf. durch eine oder mehrere Gruppen substituiert ist, die aus den verzweigten oder unverzweigten Resten Alkyl, Alkenyl oder Alkoxy mit 1 bis 6 Kohlenstoffatomen ausgewählt sind,
oder B einen Rest NR₃₄R₃₅ darstellt, in dem R₃₄ und R₃₅ unabhängig voneinander ein Wasserstoffatom oder einen verzweigten oder unverzweigten Alkylrest mit 1 bis 6 Kohlenstoffatomen darstellen oder R₃₄ und R₃₅ mit dem Stickstoffatom einen nicht-aromatischen Heterocyclus mit fünf bis sechs Kettengliedern bilden, wobei die Elemente der Kette aus einer Gruppe ausgewählt sind, die aus -CH₂-, -NH-, -O- oder -S- besteht;
X eine Bindung oder einen Rest -(CH₂)ₘ-, -(CH₂)ₘ-CO, -O-(CH₂)ₘ-, -S-(CH₂)ₘ-, -NR₃₆-(CH₂)ₘ-, -CO-NR₃₆-, -O-(CH₂)ₘ-CO-, -S-(CH₂)ₘ-CO-, -NR₃₆-(CH₂)ₘ-CO-, -(CH₂)ₘ-C(OH) (CH₃)-CO-, -CH=CH- oder -CH=N- darstellt;
Y eine Bindung oder einen Rest -(CH₂)ₙ- oder -(CH₂)ᵣ-Q-(CH₂)ₛ- darstellt, wobei Q einen Rest Piperazin, Homopiperazin, 2-Methylpiperazin, 2,5-Dimethylpiperazin, Piperidin, 1,2,3,6-Tetrahydropyridin, Pyrrolidin, Acetidin, Thiazolidin oder einen gesättigten kohlenstoffhaltigen Cyclus mit 3 bis 7 Kettengliedern darstellt;
Φ eine Bindung oder einen Rest (CH₂)ₚ-O-CH₂)_{q}-, -(CH₂)ₚ-S-(CH₂)_{q}-, -(CH₂)ₚ-NR₃₇-(CH₂)_{q}-, -(CH₂)ₚ-CO-NR₃₇-(CH₂)_{q}- oder -CO-(CH₂)ₚ-NR₃₇-(CH₂)_{q}- darstellt;
R₃₆ und R₃₇ unabhängig voneinander ein Wasserstoffatom, einen verzweigten oder unverzweigten Alkylrest mit 1 bis 6 Kohlenstoffatomen oder einen Rest -CO-R₃₈ darstellen, in dem R₃₈ einen Alkyl- oder einen verzweigten oder unverzweigten Alkoxyrest mit 1 bis 6 Kohlenstoffatomen darstellt;
R₃₉ ein Wasserstoffatom oder einen Alkyl- oder einen verzweigten oder unverzweigten Alkoxyrest mit 1 bis 6 Kohlenstoffatomen darstellt;
m, n, p, q, r und s ganze Zahlen von 0 bis 6 sind;
oder die Salze der Produkte der allgemeinen Formel (I').

2. Produkt nach Anspruch 1, **dadurch gekennzeichnet, daß** R₃₁ einen Methyl-, Benzyl- oder Naphthylmethylrest darstellt.

3. Produkt nach Anspruch 1, **dadurch gekennzeichnet, daß** es aus den Verbindungen
- 3-[3-{[Amino(2-thienyl)methyliden]amino}-benzyl)amino]-N-(1-methyl-2,3-dihydro-1H-indol-5-yl)propanamid;
- 3-[(3-{[Amino(2-thienyl)methyliden]amino}-benzyl)amino]-N-(1-benzyl)-2,3-dihydro-1H-indol-5-yl)propanamid;
- 3 [(3-{[Amino(2-thienyl)methyliden]amino}benzyl)amino]-N-[1-(1-naphthylmethyl)-2,3-dihydro-1H-indol-5-yl]propanamid;
- 4-(4-{[Amino(2-thienyl)methyliden]amino}phenyl)-N-{1-[3-(dimethylamino)propyl]-2,3-dihydro-1H-indol-5-yl}butanamid;
- 3-[(5-{[Amino(2-thienyl)methyliden]amino}-2-methoxybenzyl)amino]-N-[1-(1-naphthylmethyl)-2,3-dihydro-1H-indol-5-yl]propanamid;
und deren Salzen ausgewählt ist.

4. Die Verbindungen der allgemeinen Formel (IS') 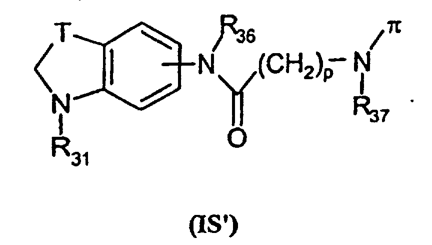 in der
π ein Wasserstoffatom oder eine Schutzgruppe vom Typ Carbamat darstellt;
R₃₆ und R₃₇ unabhängig voneinander ein Wasserstoffatom, einen verzweigten oder unverzweigten Alkylrest mit 1 bis 6 Kohlenstoffatomen oder einen Rest -CO-R₃₈ darstellen, in dem R₃₈ einen Alkyl- oder einen verzweigten oder unverzweigten Alkoxyrest mit 1 bis 6 Kohlenstoffatomen darstellt;
T einen Rest -(CH₂)ₖ- darstellt, wobei k 1 oder 2 darstellt;
R₃₁ einen verzweigten oder unverzweigten Alkylrest mit 1 bis 6 Kohlenstoffatomen oder Arylalkyl, Diarylalkyl, *bis*-Arylalkyl, Aminoalkyl, Alkylaminoalkyl oder Dialkylaminoalkyl darstellt, deren Alkylreste verzweigt oder unverzweigt sind und 1 bis 6 Kohlenstoffatome zählen;
und p eine ganze Zahl von 0 bis 6 ist,
in Form von neuen Industrieprodukten.

5. Ein Produkt der allgemeinen Formel (I') nach Anspruch 1 oder ein pharmazeutisch verträgliches Salz dieses Produkts in Form eines Medikaments.

6. Pharmazeutische Zusammensetzung; die als Wirkstoff mindestens ein Produkt nach Anspruch 1 oder ein pharmazeutisch verträgliches Salz dieses Produkts enthält.

7. Verwendung eines Produkts der allgemeinen Formel (I') nach Anspruch 1 oder eines pharmazeutisch verträglichen Salzes dieses Produkts für die Herstellung eines Medikaments, das gleichzeitig eine Wirkung der Hemmung der NO-Synthase und der Hemmung der Lipidperoxidation besitzt.

8. Verwendung eines Produkts der allgemeinen Formel (I') nach Anspruch 1 oder eines pharmazeutisch verträglichen Salzes dieses Produkts für die Herstellung eines Medikaments für die Behandlung von kardiovaskulären und zerebrovaskulären Krankheiten.

9. Verwendung eines Produkts der allgemeinen Formel (I') nach Anspruch 1 oder eines pharmazeutisch verträglichen Salzes dieses Produkts für die Herstellung eines Medikaments für die Behandlung von Störungen des Zentralnervensystems oder des peripheren Nervensystems.

10. Verwendung nach Anspruch 9, **dadurch gekennzeichnet, daß** das hergestellte Medikament für die Behandlung von neurodegenerativen Erkrankungen bestimmt ist.
